# EUROPEAN PATENT SPECIFICATION

(11) **EP 2 793 637 B1**
(45) Date of publication and mention of the grant of the patent: **14.10.2015**
(21) Application number: 12799222.0
(22) Date of filing: 13.12.2012
(51) Int. Cl.: A45D 19/00, A45D 19/02, A61Q 5/06, A61K 8/72

(54) **APPLICATION DEVICE COMPRISING A COMPOSITION BASED ON A HYDROPHOBIC FILM-FORMING POLYMER AND A VOLATILE SOLVENT, AND PROCESS FOR TREATING KERATIN FIBRES USING THE SAME**
APPLIKATIONSVORRICHTUNG MIT EINER ZUSAMMENSETZUNG AUF BASIS EINES HYDROPHOBEN FILMBILDENDEN POLYMERS UND EINES FLÜCHTIGES LÖSUNGSMITTELS SOWIE VERFAHREN ZUR BEHANDLUNG VON KERATINFASERN DAMIT
DISPOSITIF D'APPLICATION CONTENANT UNE COMPOSITION À BASE DE POLYMÈRE FILMOGÈNE HYDROPHOBE ET DE SOLVANT VOLATIL, ET PROCÉDÉ DE TRAITEMENT DE FIBRES KÉRATINIQUES UTILISANT CE DISPOSITIF

(30) Priority: 20.12.2011 FR 1161999; 01.02.2012 US 201261593458 P
(43) Date of publication of application: 29.10.2014
(73) Proprietor: L'OREAL, 75008 Paris (FR)
(72) Inventor: TEBOUL, Karen, F-94160 St Mande (FR)
(74) Representative: Duvert, Sandra
(86) International application number: PCT/EP2012/075419
(87) International publication number: WO 2013/092380

(56) References cited:
- WO-A1-2010/071777
- WO-A2-2007/132088
- US-A- 2 463 611
- US-A- 5 961 665
- US-A1- 2011 005 546

## Description

The present invention relates to a device for applying a composition for treating keratin fibres, comprising a hydrophobic film-forming polymer and a volatile solvent, and also to a process for treating keratin fibres using such a device.

The hair is generally damaged and embrittled by the action of external atmospheric agents such as light and bad weather, and by mechanical or chemical treatments, such as brushing, combing, bleaching, permanent-waving and/or dyeing. As a result, the hair is often difficult to manage and in particular is difficult to disentangle or style, and a head of hair, even of lush hair, has difficulty in maintaining an attractive style due to the fact that the hair lacks vigour, volume and liveliness.

This degradation to the hair is increased, moreover, by the repetition of permanent hair colouring treatments, which involves applying to the hair one or more dye precursors and an oxidizing agent.

Thus, to overcome this, it is now common practice to use styling products that allow the hair to be conditioned by especially giving it body, mass or volume.

These styling products are generally cosmetic hair compositions comprising one or more polymers with high affinity for the hair, which often have the function of forming a film on the hair surface in order to modify its surface properties and especially to condition it or to give it particular optical properties.

One drawback associated with the use of these hair compositions lies in the fact that the cosmetic effects imparted by such compositions have a tendency to disappear, especially at the first shampoo wash.

In order to overcome this drawback, it may be envisaged to increase the remanence of the deposit of polymers by directly performing free-radical polymerization of certain monomers on the hair. However, the treatments thus obtained result in the degradation of the fibre and the hair thus treated is generally difficult to disentangle.

It is also known practice to dye keratin fibres with pigments. The compositions may contain a pigment and a film-forming polymer. In order to improve the remanence of the coloured or uncoloured deposit on keratin fibres, it is known practice to apply a composition comprising a pressure-sensitive adhesive silicone copolymer, more commonly known as a BioPSA. The feel obtained with these copolymers is generally tacky. Hair treated with this type of composition has a slightly coarse and not entirely natural feel. Furthermore, the application of this type of composition is time-consuming and requires a step of total drying that is obligatory in order to obtain remanent coating.

Moreover, hair treatment products (gels, shampoos or products for caring for or conditioning the hair) are generally perfumed, but have very limited remanence of the perfume on the hair, the perfume generally fading after a few minutes or, in the best of cases, after a few hours.

Devices for applying a product onto a strand of hair or fibers are known, for example, from US 2011 005 546 or US 5 961 665.

Thus, the aim of the present invention is to develop a device for treating keratin fibres, and in particular human keratin fibres such as the hair, which is easy to use and which can produce coloured or uncoloured coatings that are remanent with respect to shampoo and to the various attacking factors to which the hair may be subjected, especially blow-drying and perspiration, while at the same time showing better tolerance towards fatty substances such as sebum and not developing any tacky nature. In particular, the aim of the present invention is to obtain coloured or uncoloured coatings, which are not tacky, easy to use and resistant to external agents, and which respect the integrity of keratin fibres.

Thus, the aim of the present invention is to propose a device for facilitating the application of a hair treatment composition; this composition also giving access to a coating that is remanent to shampoo and to the various attacking factors to which keratin fibres may be subjected, without degrading the said fibres; this coating moreover being homogeneous and smooth on the keratin fibres, leaving them perfectly individualized.

This aim is achieved with the present invention, one subject of which is thus a device (1) for applying a keratin fibre treatment composition, comprising:
i) an applicator means (2) that is capable of retaining an amount of the treatment composition (P) in a container (20),
ii) a holding member (4) that can engage with the applicator means (2), in order, during a longitudinal movement of the device (1) relative to a lock of keratin fibres, to hold the said lock in engagement with the applicator means (2) so as to enable the lock to be coated with the treatment composition (P),
iii) the said applicator means (2) comprises an applicator tip (30) mounted on the container (20) and comprising an outlet orifice (31) equipped with an opening/closing member (32), which, in a first position, closes off the said outlet orifice, and which, in a second position, at least partially releases the said outlet orifice, the passage from the first to the second position taking place in response to a stress exerted on the opening/closing member (32) by engaging the said lock between the holding member and this opening/closing member (32);
iv) the treatment composition (P) comprising at least one hydrophobic film-forming polymer and at least one volatile solvent.

A subject of the invention is also a process for treating keratin fibres, comprising the application to the fibres of a treatment composition (P) contained in a device as described previously; the application optionally being followed by drying of the fibres.

The expression "at least one" means "one or more".

The expression "comprising a" should be understood as meaning "comprising at least one", unless otherwise specified.

By using such a device, the treatment composition it contains can be applied simply, without risk of running of the product, whether in the case of a self-application or of a treatment performed by another person. The application is quick and efficient, without requiring the use of additional accessories, and the impregnation of the fibres is homogeneous, irrespective of their length.

Moreover, the composition applied produces a coating that is remanent with respect to shampoo, while at the same time preserving the physical qualities of the keratin fibre. Such a coating is, in particular, resistant to the external attacking factors to which the fibres may be subjected, such as blow-drying and perspiration. It makes it possible in particular to obtain a smooth, uniform deposit. Moreover, it has been observed, surprisingly, that the fibres remain perfectly individualized and can be styled without problem, and that the styling properties afforded to the fibres are shampoo-fast. With the process of the invention, a remanent coating is obtained without it being necessary to dry the hair with a hairdryer. The hair after application is left in the open air, and after a few seconds the remanent coating is formed.

The term "individualized fibres" means fibres which, after application of the composition and drying, are not stuck together (or are separate from each other) and therefore do not form clumps, since the coating is formed around virtually every fibre.

### Device

The device according to the invention is most particularly suitable for applying a treatment product to one or more fibres.

The application may be performed locally onto a whole head hair by working successively on at least all the apparent locks of the said head of hair.

Preferably, the holding member is mobile relative to the applicator means.

According to one variant, the holding member and the applicator means are linked via a connecting means and form a single piece.

According to another variant, the holding member and the applicator means form two separate pieces.

Advantageously, the container comprising the composition comprises an annular narrowing to make it easy to hold between two fingers.

In accordance with one particular embodiment, the applicator tip comprises a surface that is capable of storing and releasing the treatment composition.

Preferably, the area of the holding member can be selected to be greater than or equal to the area of the applicator end piece.

Advantageously, the holding member comprises combing means.

Preferably, the passage from the first to the second position takes place in response to a stress exerted axially on the opening/closing member.

Preferably, the applicator means and the holding member can be selected to be symmetrical to one another in relation to an axis bisecting the connecting means.

Reference may be made to the figures, which are, however, presented merely as a guide and are in no way limiting on the device.

The figures show:
**Figure 1****:** an exploded perspective view of a device according to the invention for applying a haircare product to locks,
**Figure 2****:** shows an exploded perspective view of another device according to the invention for applying a haircare product to locks,
**Figure 3****:** shows an exploded perspective view of the top part of the applicator,
**Figure 4****:** shows a top view of the applicator in the released position (second position),
**Figure 5****:** shows a top view of the applicator in the closed-off position (first position),
**Figure 6****:** shows a perspective view of an opening/closing element in the released position (second position),
**Figure 7****:** shows a perspective view of an opening/closing element in the closed-off position (first position),
**Figure 8****:** shows a front view of the skirt of the applicator,
**Figure 9****:** shows a top view of the skirt of the applicator,
**Figure 10****:** shows a perspective view of a device according to the invention in one piece before use,
**Figure 11****:** shows a perspective view of a device according to the invention in two pieces before use,
**Figure 12****:** shows a perspective view of a device according to the invention in one piece during use.

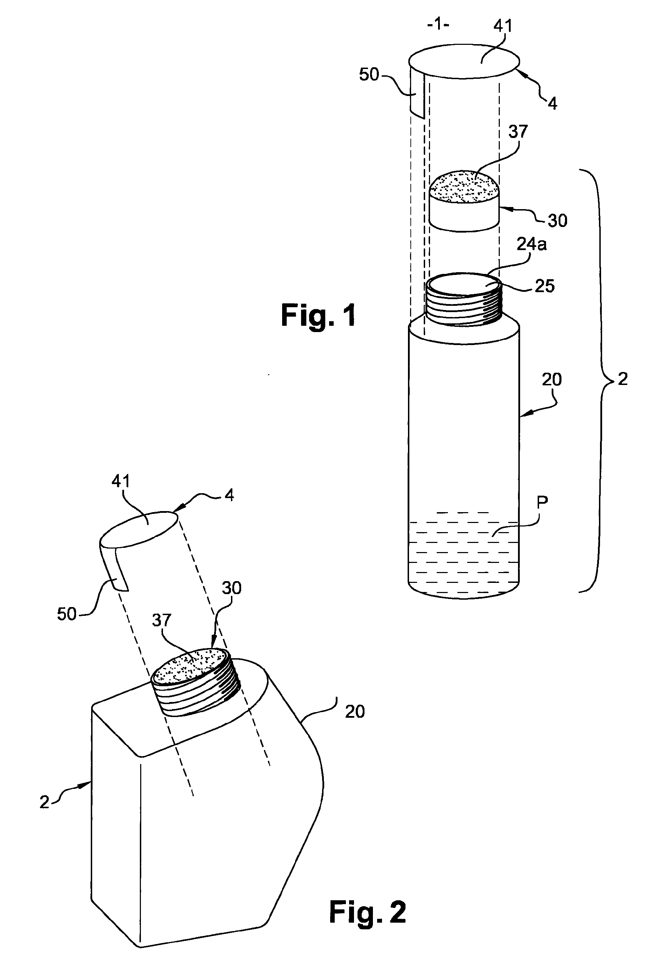

The device represented in figures 1 and 2 comprises an applicator means 2 containing the composition that is useful in the invention and a holding member 4. The application device 2 is known per se and described in particular in US 5 961 665.

The application device 2 comprises a container 20 in the form of a small supple or rigid bottle. Alternatively, a bottle made of thermoplastic material, for example PET, may be used. The bottle has a capacity of, for example, 6 ml. The bottle comprises a side wall in the form of a cylinder of revolution, one end of which is closed by an end wall 23. The second end is formed by a portion having a narrowed diameter, which ends at a free edge 24a defining an opening 25.

An applicator end piece 30 is provided to be fitted onto the bottle and to be snap-fastened or screwed onto the opening 25 in the bottle. The end piece is in the form of an approximately cylindrical shell having a constant circular diameter over a large part of its length. It could have any other form, for example a frustoconical form, becoming progressively smaller until it defines a circular portion. The tip 30 has, for example, a diameter of about 15 mm. Axial ribs may be provided on the inside wall of the shell. They may have a radial indentation which, in the fitted position of the shell, are housed in the opening 25 in the bottle, thereby allowing the shell to be snap-fastened to the bottle. Alternatively, it is possible to provide for the inside wall of the shell to be provided with a thread provided to engage with a thread provided on the neck of the bottle.

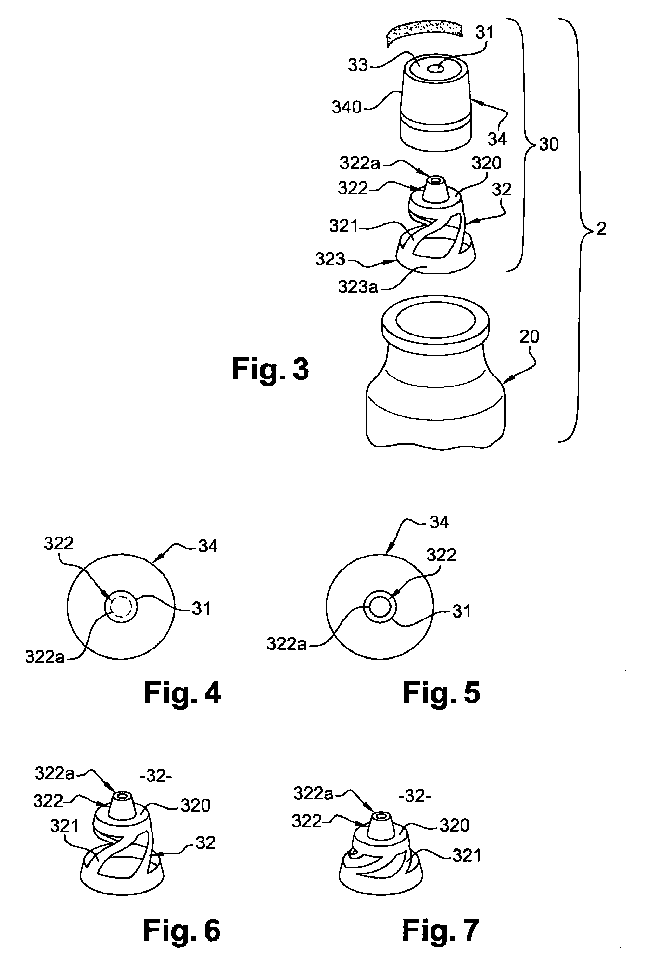

As can be seen in Figures 3 to 9, the end piece comprises a cylindrical skirt 34, which provides sealing between the opening 25 in the bottle and the outlet orifice 31. The skirt extends approximately axially from the cylindrical or frustoconical portion of the shell, around the seat 33, as far as a free edge 340 that forms a sealing lip. In the fitted position of the applicator end piece 30, the lip 340 comes into sealing abutment against the upper edge 24a. Thus, the sealing skirt does not overlap the bottle laterally such that the presence of the skirt does not affect the lateral space requirement of the shell. A radial protrusion 341 is provided on the inside wall of the skirt 34 for the abutment of the opening/closing element 32. This is a continuous circular bead. It is quite clear that a discontinuous bead formed for example of portions that are angularly spaced apart can be used for the abutment of the closure element.

The end piece is advantageously obtained by moulding a single piece of a preferably thermoplastic material, in particular polyethylene, polypropylene, polyethylene terephthalate, polyvinyl chloride, polyamide.

The opening/closing element 32 comprises a spring 321 which is formed by helical coils, the lower part of which is held in the material of the mounting ring 323. The lower edge 323a of the mounting ring 323 is in abutment against the radial protrusion 341 of the skirt 34. The upper part of the spring is held in the material of a hollow shaped body which ends with a frustoconical region 320 that is provided to come into abutment against the seat 33, thereby forming a valve. The frustoconical region is extended by a stud 322 having a circular cross section, the end 322a of which can be slightly rounded in order not to damage the surface to be treated, with which it comes into direct contact. The diameter of the stud is less than the diameter of the dispensing orifice in order that the stud can slide easily through the orifice 31, allowing product for dispensing to pass through. In the rest position, the stud 322 protrudes out of the orifice such that its end 322a can be placed in direct contact with a surface to be treated or in indirect contact via a membrane 37.

The opening/closing element 32 is likewise obtained by moulding a single piece of a preferably thermoplastic material, in particular polyethylene, polypropylene, polyethylene terephthalate, polyvinyl chloride, polyamide.

In the fitted position of the opening/closing element, the lower edge 323a of the mounting ring is in abutment against the radial protrusion and the spring 321 is slightly compressed, in order to keep the frustoconical region 320 in sealed abutment against the seat 33 so as to prevent any leakage of product through the orifice 31. When the stud is brought into contact with a surface to be treated, a force is exerted on the stud, said force being transmitted to the spring so as to compress it. The stud is thus inserted into the orifice 31 and the frustoconical portion disengages from the seat 33, as is shown in Figures 3 to 7.

The membrane 37 can be porous or non-porous, be supported on the end piece 30, have for example at least one orifice for dispensing product passing through it, and be covered externally at least partially with a flocked surface, the membrane defining, with the end piece 30, an internal space that can contain product to be dispensed.

The membrane may be flexible and/or mounted on the end piece 30 such that it can be deformed and/or moved in relation to the support and/or to the reservoir, leading to a reduction in volume of the internal space of at least 0.1 ml.

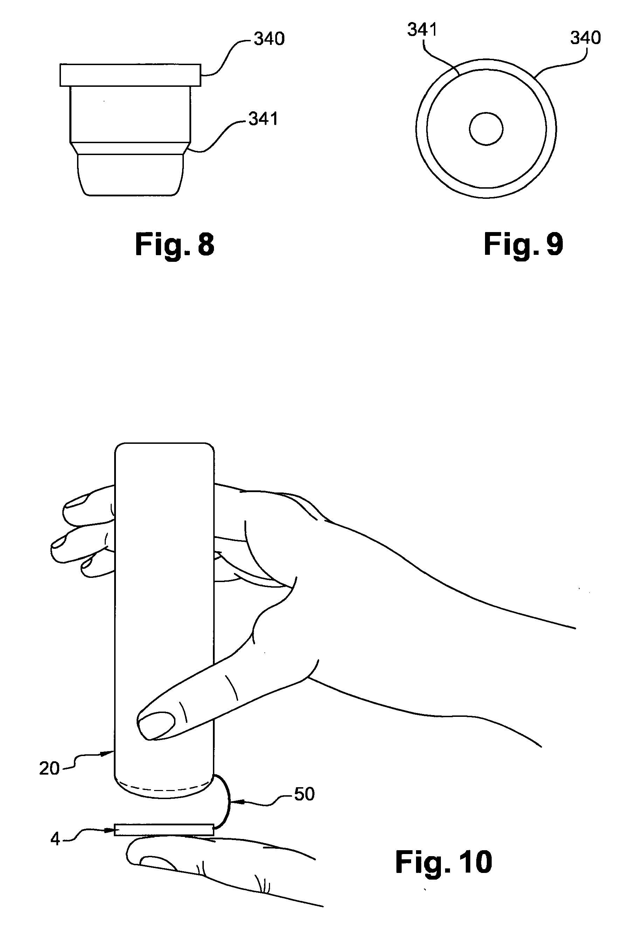

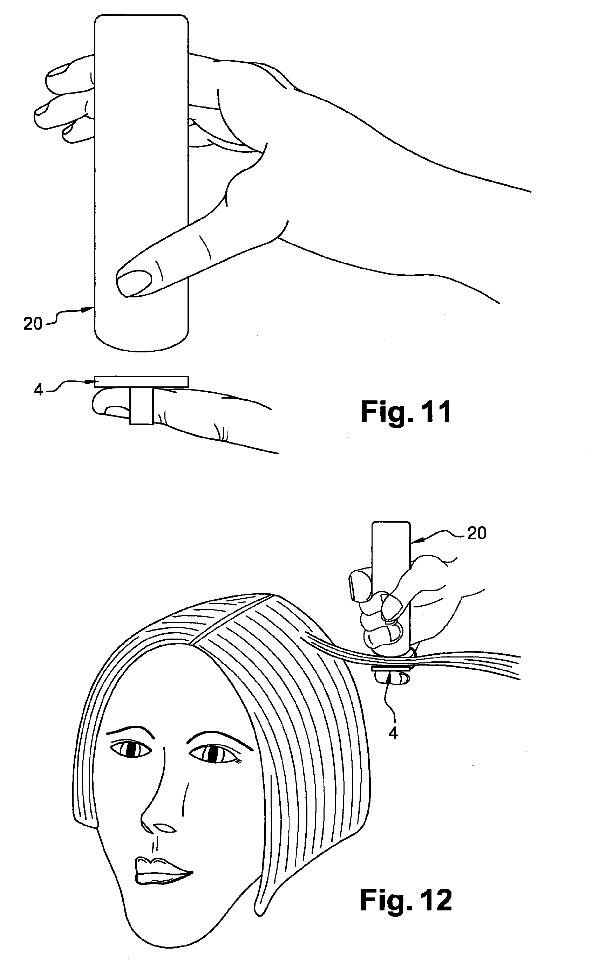

As is shown in Figures 10 and 11, in order to use the device, the user takes hold of the bottle, turns it over and applies the end piece to the lock. It thus suffices for him to prolong his hand movement in order to depress the stud 322 and disengage the frustoconical region 320 from the seat 33 in order at least partly to clear the orifice 31 (Figure 3). To do this, he places his thumb in contact with the holding member 4. The product exits by gravity through the orifice. Since the orifice has a small diameter, the product flows onto the lock in a very localized manner, thereby making it possible to apply the product in a highly precise manner. Next, he moves the device, clamping the lock. As soon as the user moves the dispenser away from the lock or no longer exerts any pressure with his thumb and the force exerted on the stud stops, the spring returns to its rest position and the frustoconical portion comes back into abutment against the seat. The product thus stops flowing and air is drawn into the bottle before the valve is closed.

According to a particular embodiment, the holding member 4 comprises a circular cover 41 having a diameter greater than that of the membrane 37. It also comprises a connecting strip 50 made of flexible material, which is chosen so as to elastically lengthen by the distance necessary for positioning the lock. This strip 50 may be made of elastomer, in particular thermoplastic elastomer, such as a polyethylene elastomer. The strip 50 may be moulded in one piece with the cover 41 or be connected to the cover 41 by adhesive bonding or welding.

The connecting means 50 may consist in particular of film hinges or hinges of the pinned hinge type, or having an element supported by one of said first or second parts, said element being mounted in rotation inside a recess formed in the other of said end wall or said cover 41.

The cover 41 may have a shape chosen from the following list: circular, non-circular, especially oblong, oval, elliptical, polygonal, especially square, rectangular, reniform, crenellated or star-shaped, with one or more grooves.

The cover 41 and the membrane 50 may be made of different materials. At least one of them may be made of a thermoplastic material, especially of a material chosen from the group consisting of: PE, PP, POM, PA, PET, PBT.

The cover 41 may comprise at least one of the following applicator members: bundle of bristles, felt, flock coating, foam or other applicator members such as studs or teeth. These applicator elements are disposed on the surface of the cover 41, coming into contact with the lock.

These applicator elements can be disposed in the form of one or more lines or be distributed in a periodic pattern. The applicator element may be for example a comb or a brush.

The applicator members may especially be attached by bonding or stapling, or by overmoulding on the part 41. The applicator elements may also have a flocked tip.

The holding member 4 may have a gripping member for gripping the device with a predefined orientation. The gripping member may have at least one region for receiving a finger, in particular a flattened portion or a hole, which extends generally approximately parallel to an axis having a greater dimension than a cross section of the flexible part.

According to an alternative which is not shown, the geometric articulation axis is defined by a single film hinge, while the elastic return is ensured by two lateral connecting strips disposed on either side of the film hinge. The choice of such and such a configuration depends largely on the cross section of the container.

When the connecting member 4 does not comprise a connecting means 50, the cover 41 can be welded, moulded or adhesively bonded to a ring or to a fingerstall, into which at least one finger can be inserted. In this case, the ring or the fingerstall threads onto the thumb of one hand of the user and the applicator means 2 is clamped between the other fingers of the same hand. The lock to be coated is disposed next to the membrane 37 of the applicator means 2. The thumb is folded back in order to pinch the lock between the membrane 37 and the cover 41. Manual pressure is exerted along the axis of the container 20 and at right angles to the membrane 37. The user moves his hand away from the head, maintaining this pressure. He releases the pressure to stop the application to the lock.

The applicator means 2 may comprise a foam tip that may be replaced with a tip of roll-on type (the roll-on may be a sphere, a cylinder or an ovoid shape such as a rugby ball).

With the device according to the invention, the hand (for the styler or a person) may be positioned in the most appropriate manner at the user's choice, with or without the annular narrowing for making it easy to hold the applicator means 2.

### Composition for treating keratin fibres

For the purposes of the invention, the term "polymer" means a compound corresponding to the repetition of one or more units (these units being derived from compounds known as monomers). This or these unit(s) are repeated at least twice and preferably at least 3 times.

The term "film-forming" polymer means a polymer that is capable, by itself or in the presence of an auxiliary film-forming agent, of forming a macroscopically continuous film on a support, especially on keratin materials, and preferably a cohesive film.

The term "hydrophobic polymer" means a polymer that has a solubility in water at 25°C of less than 1 % by weight.

The term "film-forming" polymer means a polymer that is capable, by itself or in the presence of an auxiliary film-forming agent, of forming a macroscopically continuous film on a support, especially on keratin materials, and preferably a cohesive film.

In one embodiment, the hydrophobic film-forming organic polymer is at least one polymer chosen from the group comprising:
- film-forming polymers that are soluble in an organic solvent medium, in particular liposoluble polymers; this means that the polymer is soluble or miscible in the organic medium and forms a single homogeneous phase when it is incorporated into the medium;
- film-forming polymers that are dispersible in an organic solvent medium, which means that the polymer forms an insoluble phase in the organic medium, the polymer remaining stable and/or compatible once incorporated into this medium. In particular, such polymers may be in the form of non-aqueous dispersions of polymer particles, preferably dispersions in silicone oils or hydrocarbon-based oils; in one embodiment, the non-aqueous polymer dispersions comprise polymer particles stabilized on their surface with at least one stabilizer; these non-aqueous dispersions are often referred to as NADs;
- film-forming polymers in the form of aqueous dispersions of polymer particles, which means that the polymer forms an insoluble phase in water, the polymer remaining stable and/or compatible once incorporated into the water, the polymer particles possibly being stabilized at their surface with at least one stabilizer. These polymer particles are often referred to as latices; in this case, the composition must comprise an aqueous phase.

Among the hydrophobic film-forming polymers that may be used in the composition of the present invention, mention may be made of synthetic polymers, of radical type or of polycondensate type, polymers of natural origin, and mixtures thereof. Hydrophobic film-forming polymers that may be mentioned in particular include acrylic polymers, polyurethanes, polyesters, polyamides, polyureas, cellulose-based polymers such as nitrocellulose, silicone polymers, polyamide polymers and copolymers, and polyisoprenes.

The film-forming polymer may be chosen from the film-forming polymers described in patent application WO 04/028 487.

The hydrophobic film-forming polymer may especially be chosen from:
a) homopolymers and copolymers of olefins; cycloolefins; butadiene; isoprene; styrene; vinyl ethers, esters or amides; (meth)acrylic acid esters or amides containing a linear, branched or cyclic C₁-C₂₀ alkyl group, a C₆-C₁₀ aryl group or a C₂-C₆ hydroxyalkyl group.

Such homopolymers and copolymers may be obtained from monomers chosen from the group constituted by isooctyl (meth)acrylate, isononyl (meth)acrylate, 2-ethylhexyl (meth)acrylate, lauryl (meth)acrylate, isopentyl (meth)acrylate, n-butyl (meth)acrylate, isobutyl (meth)acrylate, ethyl (meth)acrylate, methyl (meth)acrylate, tert-butyl (meth)acrylate, tridecyl (meth)acrylate, stearyl (meth)acrylate, hydroxyethyl (meth)acrylate, 2-hydroxypropyl (meth)acrylate, benzyl acrylate and phenyl acrylate, or mixtures thereof. Amides of the acid monomers that may be mentioned include (meth)acrylamides, and especially N-alkyl(meth)acrylamides, in particular of a C₂-C₁₂ alkyl, such as N-ethylacrylamide, N-t-butylacrylamide and N-octylacrylamide; N-di(C₁-C₄)alkyl(meth)acrylamides and perfluoroalkyl (meth)acrylates. The above polymers may also contain as monomers small amounts of an unsaturated carboxylic or sulfonic acid such as acrylic acid, methacrylic acid or AMPS, on condition that the overall nature of the polymer remains hydrophobic.

As other vinyl monomers that may be used, mention may also be made of:
- N-vinylpyrrolidone, vinylcaprolactam, vinyl-N-(C₁-C₆)alkylpyrroles, vinyloxazoles, vinylthiazoles, vinylpyrimidines and vinylimidazoles,
- olefins such as ethylene, propylene, butenes, isoprene and butadienes.

The vinyl polymer may be crosslinked using one or more difunctional monomers, especially comprising at least two ethylenic unsaturations, such as ethylene glycol dimethacrylate or diallyl phthalate.

Mention will be made, for example, of the alkyl acrylate/cycloalkyl acrylate copolymer sold by Phoenix Chem. under the name Giovarez AC-5099 ML, the acrylates/C12-22 alkyl methacrylate copolymer sold by Röhm & Haas under the name Soltex OPT and vinylpyrrolidone copolymers, such as copolymers of a C₂-C₃₀ alkene, such as a C₃-C₂₂ alkene, and combinations thereof. As examples of VP copolymers that may be used in the invention, mention may also be made of the VP/vinyl laurate copolymer, the VP/vinyl stearate copolymer, the butylated polyvinylpyrrolidone (PVP) copolymer, the VP/hexadecene copolymer sold by ISP under the name Ganex V216, the VP/eicosene copolymer sold by ISP under the name Ganex V220, the VP/triacontene copolymer or the VP/acrylic acid/lauryl methacrylate copolymer. Mention may also be made of the copolymers whose CTFA name (4th edition, 1991) is octylacrylamide/acrylates/butylaminoethyl methacrylate copolymer, such as the products sold under the name Amphomer® or Lovocryl® 47 by the company National Starch, and also the copolymers whose CTFA name is acrylates/octylacrylamide copolymer, such as the products sold under the name Dermacryl® LT or Dermacryl® 79 by the company National Starch.

Particular polymers that may be mentioned include:
i) polymers bearing fluoro groups belonging to one of the classes described in the above text, in particular the Fomblin products described in patent US 5 948 393, and the copolymers of alkyl (meth)acrylate/perfluoroalkyl (meth)acrylate described in patents EP 0 815 836 and US 5 849 318.
ii) polymers or copolymers resulting from the polymerization or copolymerization of an ethylenic monomer, comprising one or more ethylenic bonds, which are preferably conjugated (or dienes). As polymers or copolymers resulting from the polymerization or copolymerization of an ethylenic monomer, it is possible to use vinyl, acrylic or methacrylic copolymers.

In one embodiment, the film-forming polymer is a block copolymer comprising at least one block consisting of styrene units or styrene derivatives (for example methylstyrene, chlorostyrene or chloromethylstyrene). The copolymer comprising at least one styrene block may be a diblock or triblock copolymer, or even a multiblock copolymer, in starburst or radial form. The copolymer comprising at least one styrene block may also comprise, for example, an alkylstyrene (AS) block, an ethylene/butylene (EB) block, an ethylene/propylene (EP) block, a butadiene (B) block, an isoprene (I) block, an acrylate (A) block, a methacrylate (MA) block or a combination of these blocks. The copolymer comprising at least one block constituted of styrene units or styrene derivatives may be a diblock or triblock copolymer, and in particular of the polystyrene/polyisoprene or polystyrene/polybutadiene type, such as those sold or manufactured under the name Luvitol HSB by BASF and those of the polystyrene/copoly(ethylene-propylene) type or alternatively of the polystyrene/copoly(ethylene/butylene) type, such as those sold or manufactured under the brand name Kraton by Shell Chemical Co. or Gelled Permethyl 99A by Penreco.

Mention may be made, for example, of Kraton G1650 (SEBS), Kraton G1651 (SEBS), Kraton G1652 (SEBS), Kraton G1657X (SEBS), Kraton G1701X (SEP), Kraton G1702X (SEP), Kraton G1726X (SEB), Kraton D-1101 (SBS), Kraton D-1102 (SBS), Kraton D-1107 (SIS), Gelled Permethyl 99A-750, Gelled Permethyl 99A-753-58 (mixture of star block polymer and of triblock polymer), Gelled Permethyl 99A-753-59 (mixture of star block polymer and of triblock polymer), Versagel MD 970 and Versagel MD 960 from Penreco (mixture of star polymer and of triblock polymer in isododecane).

Styrene-methacrylate copolymers may also be used, such as the polymers sold under the references OS 129880, OS 129881 and OS 84383 from Lubrizol (styrene-methacrylate copolymer).

In one embodiment, the film-forming polymer is chosen from copolymers of vinyl ester (the vinyl group being directly connected to the oxygen atom of the ester group and the vinyl ester having a saturated, linear or branched hydrocarbon-based radical of 1 to 19 carbon atoms bonded to the carbonyl of the ester group) and of at least one other monomer which is chosen from vinyl esters (other than the vinyl ester already present), α-olefins (containing from 8 to 28 carbon atoms), alkyl vinyl ethers (in which the alkyl group contains from 2 to 18 carbon atoms) or allylic or methallylic esters (containing a linear or branched saturated hydrocarbon-based radical of 1 to 19 carbon atoms, bonded to the carbonyl of the ester group).

These copolymers may be partially crosslinked using crosslinking agents, which may be either of the vinyl type or of the allylic or methallylic type, such as tetraallyloxyethane, divinylbenzene, divinyl octanedioate, divinyl dodecanedioate, and divinyl octadecanedioate.

Examples of these copolymers that may be mentioned include the following copolymers: vinyl acetate/allyl stearate, vinyl acetate/vinyl laurate, vinyl acetate/vinyl stearate, vinyl acetate/octadecene, vinyl acetate/octadecyl vinyl ether, vinyl propionate/allyl laurate, vinyl propionate/vinyl laurate, vinyl stearate/1-octadecene, vinyl acetate/1-dodecene, vinyl stearate/ethyl vinyl ether, vinyl propionate/cetyl vinyl ether, vinyl stearate/allyl acetate, vinyl 2,2-dimethyloctanoate/vinyl laurate, allyl 2,2-dimethylpentanoate/vinyl laurate, vinyl dimethylpropionate/vinyl stearate, allyl dimethylpropionate/vinyl stearate, vinyl propionate/vinyl stearate, crosslinked with 0.2% divinylbenzene, vinyl dimethylpropionate/vinyl laurate, crosslinked with 0.2% divinylbenzene, vinyl acetate/octadecyl vinyl ether, crosslinked with 0.2% tetraallyloxyethane, vinyl acetate/allyl stearate, crosslinked with 0.2% divinylbenzene, vinyl acetate/1-octadecene crosslinked with 0.2% divinylbenzene, and allyl propionate/allyl stearate, crosslinked with 0.2% divinylbenzene.
iii) polyalkenes and copolymers of C₂-C₂₀ alkenes, in particular polybutene.
iv) polycondensates

Among the polycondensates that may be mentioned are nonionic polyurethanes, polyurethane-acrylics, polyurethane-polyvinylpyrrolidones, polyester-polyurethanes, polyether-polyurethanes, polyureas and polyurea-polyurethanes, and mixtures thereof.

The polyurethanes may be, for example, a copolymer of aliphatic, cycloaliphatic or aromatic polyurethane or of polyurea-polyurethane.

The polyurethanes as defined in the invention may also be obtained from branched or unbranched polyesters or from alkyds comprising mobile hydrogens that are modified by means of a polyaddition with a diisocyanate and an organic difunctional (for example dihydro, diamino or hydroxyamino) co-reagent.

Mention may also be made of polyesters, polyesteramides, fatty-chain polyesters, polyamides and epoxyester resins.

The polyesters may be obtained, in a known manner, by polycondensation of aliphatic or aromatic diacids with aliphatic or aromatic diols or with polyols. Succinic acid, glutaric acid, adipic acid, pimelic acid, suberic acid or sebacic acid may be used as aliphatic diacids. Terephthalic acid or isophthalic acid, or alternatively a derivative such as phthalic anhydride, may be used as aromatic diacids. Ethylene glycol, propylene glycol, diethylene glycol, neopentyl glycol, cyclohexanedimethanol and 4,4-N-(1-methylpropylidene)bisphenol may be used as aliphatic diols.

The polyesteramides may be obtained in a manner similar to that for the polyesters, by means of the polycondensation of diacids with amino alcohols. The polyamides may be obtained in a manner similar to that for the polyesters, by means of the polycondensation of diacids with diamines.

Particular polyesters that may be mentioned include aliphatic polyesters containing C4-50 alkyl side chains or polyesters resulting from the condensation of fatty acid dimers, or alternatively polyesters comprising a silicone segment in the form of a terminal block, graft or group, as defined in patent application FR 0 113 920.

### b) Silicone compounds.

The hydrophobic film-forming polymer may also be a polymer comprising at least one silicone portion.

In the text hereinbelow, in accordance with what is generally accepted, the terms "silicone" and "polysiloxane" mean any organosilicon polymer or oligomer of linear or cyclic, branched or crosslinked structure, of variable molecular weight, obtained by polymerization and/or polycondensation of suitably functionalized silanes, and constituted essentially of a repetition of main units in which the silicon atoms are linked together via oxygen atoms (siloxane bond ≡Si-O-Si≡), optionally substituted hydrocarbon-based radicals being bonded directly via a carbon atom to the said silicon atoms. The most common hydrocarbon-based radicals are alkyl radicals, especially C₁-C₁₀ alkyl radicals, and in particular methyl, fluoroalkyl radicals, aryl radicals and in particular phenyl, and alkenyl radicals and in particular vinyl; other types of radicals which can be linked, either directly or via a hydrocarbon-based radical, to the siloxane chain are, especially, hydrogen, halogens and in particular chlorine, bromine nr fluorine, thiols, alkoxy radicals, polyoxyalkylene (or polyether) radicals and in particular polyoxyethylene and/or polyoxypropylene radicals, hydroxyl or hydroxyalkyl radicals, substituted or unsubstituted amine groups, amide groups, acyloxy or acyloxyalkyl radicals, hydroxyalkylamino or aminoalkyl radicals, quaternary ammonium groups, amphoteric or betaine groups, anionic groups such as carboxylates, thioglycolates, sulfosuccinates, thiosulfates, phosphates and sulfates, needless to say this list not being limiting in any way ("organomodified" silicones).

As hydrophobic film-forming polymers comprising at least one silicone portion, mention may be made especially of:
i) silicone resins, which are generally soluble or swellable in silicone oils.

These resins are crosslinked polyorganosiloxane polymers.
- The nomenclature of silicone resins is known under the name MDTQ, the resin being described as a function of the various siloxane monomer units it comprises, each of the letters MDTQ characterizing a type of unit.
- The letter M represents the monofunctional unit of formula (CH₃)₃SO_{1/2}, the silicon atom being bonded to only one oxygen atom in the polymer comprising this unit.
- The letter D means a difunctional unit (CH₃)₂SiO_{2/2} in which the silicon atom is bonded to two oxygen atoms.
- The letter T represents a trifunctional unit of formula (CH₃)SiO_{3/2}.
- The letter Q means a tetrafunctional unit SiO_{4/2} in which the silicon atom is bonded to four hydrogen atoms, which are themselves bonded to the rest of the polymer.

In the units M, D and T defined previously, at least one of the methyl groups may be substituted with a group R other than a methyl group, such as a hydrocarbon-based radical (especially alkyl) containing from 2 to 10 carbon atoms or a phenyl group, or alternatively a hydroxyl group.

Various resins with different properties may be obtained from these different units, the properties of these polymers varying as a function of the type of monomers (or units), of the type and number of substituted radicals, of the length of the polymer chain, of the degree of branching and of the size of the side chains.

Examples of these silicone resins that may be mentioned include:
- siloxysilicates, which may be trimethyl siloxysilicates of formula [(CH₃)₃CSiCO]ₓₓ(SiO_{4/2})_{y} (units MQ) in which x and y are integers ranging from 50 to 80,
- polysilsesquioxanes of formula (CH₃SiO_{3/2})ₓ (units T) in which x is greater than 100 and in which at least one of the methyl radicals may be substituted with a group R as defined above,
- polymethylsilsesquioxanes, which are polysilsesquioxanes in which none of the methyl radicals is substituted with another group. Such polymethylsilsesquioxanes are described in document US 5 246 694.

As examples of commercially available polymethylsilsesquioxane resins, mention may be made of those sold:
- by the company Wacker under the reference Resin MK, such as Belsil PMS MK: polymer comprising CH₃SiO_{3/2} repeating units (T units), which may also comprise up to 1% by weight of (CH₃)₂SiO_{2/2} units (D units) and having an average molecular weight of about 10 000, or
- by the company Shin-Etsu under the references KR-220L, which are compounds of units T of formula CH₃SiO_{3/2} and have Si-OH (silanol) end groups, under the reference KR-242A, which comprise 98% of units T and 2% of dimethyl units D and have Si-OH end groups, or alternatively under the reference KR-251, comprising 88% of units T and 12% of dimethyl units D and have Si-OH end groups.

Siloxysilicate resins that may be mentioned include trimethylsiloxysilicate (TMS) resins optionally in the form of powders. Such resins are sold under the reference SR1000 by the company General Electric or under the reference TMS 803 by the company Wacker. Mention may also be made of the trimethyl siloxysilicate resins sold in a solvent such as cyclomethicone, sold under the name KF-7312J by the company Shin-Etsu or DC 749 or DC 593 by the company Dow Corning.
ii) Silicone-based polyamide copolymers of the polyorganosiloxane type, such as those described in documents US-A-5 874 069, US-A-5 919 441, US-A-6 051 216 and US-A-5 981 680.
iv) Grafted silicone compounds

The composition of the invention may also contain a grafted silicone polymer. In the context of the invention, the term "grafted silicone polymer" means a polymer comprising a polysiloxane portion and a portion constituted of a non-silicone organic chain, one of the two portions constituting the main chain of the polymer, the other being grafted onto the said main chain.

The grafted silicone polymers used in the cosmetic composition according to the invention are preferentially chosen from the group constituted by polymers with a non-silicone organic backbone grafted with monomers containing a polysiloxane, and polymers with a polysiloxane backbone grafted with non-silicone organic monomers, and mixtures thereof.

The non-silicone organic monomers constituting the main chain of the grafted silicone polymer may be chosen from radical-polymerizable ethylenically unsaturated monomers, polycondensation-polymerizable monomers such as those forming polyamides, polyesters or polyurethanes, and ring-opening monomers such as those of the oxazoline or caprolactone type.

The polymers with a non-silicone organic backbone grafted with monomers containing a polysiloxane, in accordance with the invention, may be chosen from those described in patents US 4 693 935, US 4 728 571 and US 4 972 037 and patent applications EP-A-0 412 704, EP-A-0 412 707, EP-A-0 640 105 and WO 95/00578. These are copolymers obtained by radical polymerization from ethylenically unsaturated monomers and from silicone macromers containing a vinyl end group, or alternatively copolymers obtained by reaction of a polyolefin comprising functionalized groups and of a polysiloxane macromer containing an end function that is reactive with the said functionalized groups.

The copolymer containing a non-silicone organic backbone grafted with monomers containing a polysiloxane may, for example, have the following structure:

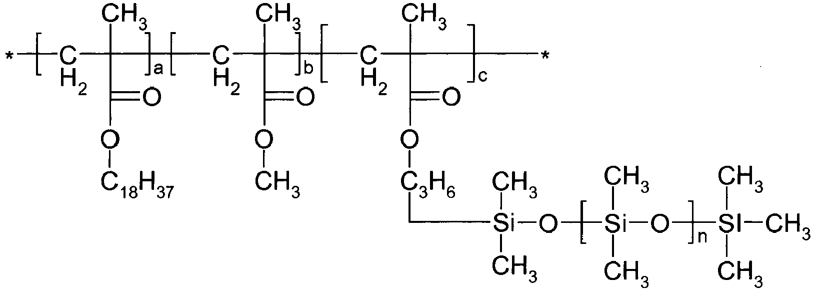

Such a polymer is sold under the name KP 561 by Shin-Etsu.

The copolymer containing a non-silicone organic backbone grafted with monomers containing a polysiloxane may also have the following structure:

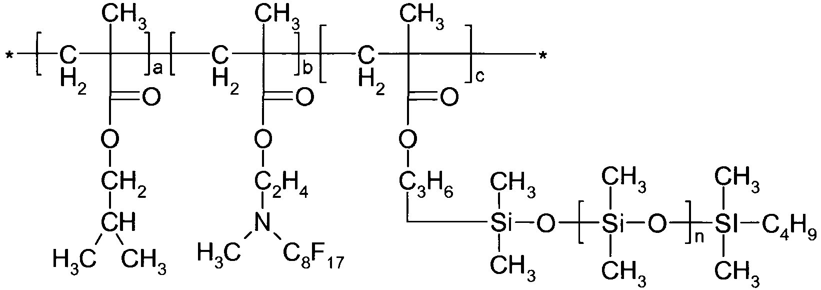

Such a polymer, Polysilicone 7, is sold under the name SA70 by 3M.

Other copolymers containing a non-silicone organic backbone grafted with monomers containing a polysiloxane may also be KP545, KP574 and KP575 sold by Shin-Etsu.

A grafted silicone compound that may also be mentioned is the isobutyl methacrylate/bishydroxypropyl dimethicone acrylate copolymer sold by Grant Industries under the name Granacrysil BMAS.

According to the present invention, the grafted silicone polymer(s), containing a polysiloxane backbone grafted with non-silicone organic monomers, comprise(s) a main silicone chain (or polysiloxane (≡Si-O-)ₙ) onto which is grafted, within the said chain and also optionally on at least one of its ends, at least one organic group not comprising silicone.

Examples of silicone polymers corresponding to the definition are especially polydimethylsiloxanes (PDMS) onto which are grafted, via a connecting chain unit of thiopropylene type, mixed polymer units of the poly(meth)acrylic acid type and of the polyalkyl (meth)acrylate type. A compound corresponding to this definition that may be mentioned is the polydimethyl/methylsiloxane containing methyl 3-thiopropyl acrylate/methyl methacrylate/methacrylic acid groups or Polysilicone-8 sold under the name VS80 by the company 3M.

Other examples of silicone polymers are especially polydimethylsiloxanes (PDMS) onto which are grafted, via a connecting chain unit of thiopropylene type, polymer units of the polyisobutyl (meth)acrylate type.

Preferably, the number-average molecular mass of the silicone polymers containing a polysiloxane backbone grafted with non-silicone organic monomers of the invention ranges from 10 000 to 1 000 000 approximately and even more preferentially from 10 000 to 100 000 approximately.

Preferably, the grafted silicone polymers are chosen from the group constituted by the copolymer of polydimethylsiloxane-grafted alkyl methacrylate, copolymers of isobutyl methacrylate, of acrylic acid and of silicone macromer, and the polydimethyl/methylsiloxane containing methyl 3-thiopropyl acrylate/methyl methacrylate/methacrylic acid groups.
v) Polyurea/urethane silicones

The copolymer of the invention may comprise, in addition to the polysiloxane/polyurea, other blocks of different units. Mention will be made in particular of polysiloxane/polyurea/polyurethane block terpolymers.

According to one variant, the copolymer contains solely one or more siloxane blocks and one or more polyurea blocks.

According to the invention, the copolymer may correspond to the general formula (I): 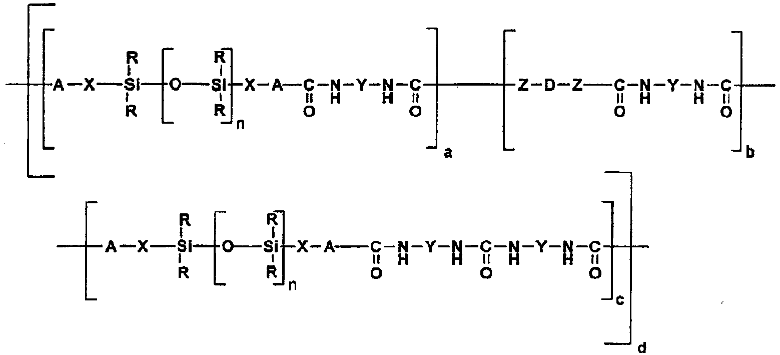 in which:
R represents a monovalent hydrocarbon-based radical, where appropriate substituted with fluorine or chlorine, containing 1 to 20 carbon atoms,
X represents an alkylene radical containing 1 to 20 carbon atoms, in which non-neighbouring methylene units may be replaced with -O- radicals,
A represents an oxygen atom or an amino radical -NR'-,
Z represents an oxygen atom or an amino radical -NR'-,
R' represents hydrogen or an alkyl radical containing 1 to 10 carbon atoms,
Y represents a divalent hydrocarbon-based radical, where appropriate substituted with fluorine or chlorine, containing 1 to 20 carbon atoms,
D represents an alkylene radical, where appropriate substituted with fluorine, chlorine, C₁-C₆ alkyl or C₁-C₆ alkyl ester, containing from 1 to 700 carbon atoms, in which non-neighbouring methylene units may be replaced with -O-, -COO-, -OCO- or -OCOO- radicals,
n is an integer from 1 to 4000,
a is a number at least equal to 1,
b is a number ranging from 0 to 40,
c is a number ranging from 0 to 30, and
d is a number greater than 0,
on condition that A represents in at least one of the units (a) an NH radical.

Preferably, R represents a monovalent hydrocarbon-based radical of 1 to 6 carbon atoms, for example methyl, ethyl, vinyl and phenyl. According to one particular embodiment, R is an unsubstituted alkyl radical.

Preferably, X represents an alkylene radical containing 2 to 10 carbon atoms. Preferably, the alkylene radical X is not interrupted.

According to one particular embodiment, the group A in all the units (b) and (c), when they are present, represents NH.

According to one particularly preferred embodiment, all the groups A represent an NH radical.

Preferably, Z represents an oxygen atom or an NH radical.

Preferably, Y represents a hydrocarbon-based radical containing from 3 to 13 carbon atoms, which is preferably unsubstituted. Preferably, Y represents a linear or cyclic aralkylene or alkylene radical.

Preferably, D represents an alkylene radical containing at least 2 and in particular at least 4 carbon atoms, and not more than 12 carbon atoms.

Also preferably, D represents a polyoxyalkylene radical, in particular a polyoxyethylene or polyoxypropylene radical containing at least 20 and in particular at least 100 carbon atoms, and not more than 800 and in particular not more than 200 carbon atoms.

Preferably, the radical D is unsubstituted.

Preferably, n represents a number equal to at least 3 and in particular at least 25, and preferably not more than 800, in particular not more than 400 and particularly preferably not more than 250.

Preferably, a represents a number greater than 50.

When b is other than 0, b preferably represents a number not greater than 50 and in particular not greater than 25.

Preferably, c represents a number not greater than 10 and in particular not greater than 5.

The copolymers of the invention may be obtained according to the polymerization processes described in patent application US 2004/0 254 325 or patent application WO 03/014 194.

According to another embodiment, the copolymer is a nonionic polysiloxane/polyurea copolymer, i.e. it does not contain any ionized or ionizable groups.

An example of a copolymer that may be mentioned is the dimethylpolysiloxane/urea copolymer, of INCI name polyurea dimethicone.

Such a polymer may be obtained especially by copolymerization of an α,ω-aminosilicone with a diisocyanate. Polymers corresponding to these characteristics are, for example, the products sold under the references Wacker-Belsil® UD 60, Wacker-Belsil® UD 80, Wacker-Belsil® UD 140 and Wacker-Belsil® UD 200 by the company Wacker.
vi) Copolymers based on silicone resin and on fluid silicone

These silicone copolymers are obtained by reacting a silicone resin and a fluid silicone.

Such copolymers are described, for example, in *Silicone Pressure Sensitive Adhesive,* Sobieski and Tangney, Handbook of Pressure Sensitive Adhesive Technology (D. Satas Ed.), von Nostrand Reinhold, New York.

In the copolymer, the silicone resin is present in a content of between 45% and 75% (relative to the total mass of silicone) and the fluid silicone is present in a content of between 25% and 55%, with the sum of the percentages of silicone resin and of fluid silicone being equal to 100. Preferably, the silicone resin is present in a content of between 55% and 65% (relative to the total mass of silicone) and the fluid silicone is present in a content of between 35% and 45%, with the sum of the percentages of silicone resin and of fluid silicone being equal to 100.

Preferably, the silicone resin according to the invention is the product of condensation of SiO₂ groups and of R₃(SiO)_{1/2} (triorganosilyl) groups for which each group R is independently selected from methyl, ethyl, propyl and vinyl radicals and for which the ratio between the SiO₂ functions and the R₃(SiO)_{1/2} functions of the silicone resin ranges from 0.6 to 0.9. Triorganosilyl groups that may be used to form the silicone resin may be trimethylsilyl, triethylsilyl, methylmethylpropylsilyl and dimethylvinylsilyl units, and mixtures thereof. The trimethylsilyl group is preferred in the context of the invention.

Preferably, the fluid silicone according to the invention is a diorganopolysiloxane containing OH end functions, having a viscosity of between 100 and 100 000 cSt at 25°C, for which the substituents of the diorganopolysiloxane are chosen independently from methyl, ethyl, propyl and vinyl radicals. The diorganosiloxanes are preferably linear polymers. Examples of diorganopolysiloxane may be, without limitation, a polydimethylsiloxane, an ethylmethylpolysiloxane, a copolymer of dimethylsiloxane and methylvinylsiloxane, and mixtures of such polymers or copolymers having OH ends. The preferred diorganopolysiloxane is a polydimethylsiloxane.

Examples of synthesis of such a copolymer are described, for example, in patent US 5 162 410 or in patent CA 711 756.

The preferred copolymers according to the invention are sold by Dow Corning under the reference BIO-PSA®, these BIO-PSA® copolymers themselves possibly being in two forms, standard or amine-compatible, and being supplied in different solvents with several silicone resin/fluid silicone ratios. Mention may be made especially of the grades 7-4400, 7-4500 and 7-4600. The Bio-PSA® that is particularly preferred according to the invention is the grade 7-4400.
vii) Reactive silicones

The hydrophobic film-forming polymer may be chosen from polymers obtained from silicone compounds X and Y that are capable of reacting together at the time of application to form the hydrophobic film-forming polymer. The term "silicone compound" means a compound comprising at least two organosiloxane units. According to one particular embodiment, compounds X and compounds Y are silicone compounds. The compounds X and Y may be amino or non-amino compounds. They may comprise polar groups that may be chosen from the following groups: -COOH, - COO⁻, -COO-, -OH, -NH₂, -NH-, -NR-, -SO₃H, -SO₃⁻, -OCH₂CH₂-, -O-CH₂CH₂CH₂-, -O-CH₂CH(CH₃)-, -NR₃⁺, -SH, -NO₂, I, Cl, Br, -CN, -PO₄³⁻, -CONH-, -CONR-, -CONH₂, - CSNH-, -SO₂-, -SO-, -SO₂NH-, -NHCO-, -NHSO₂-, -NHCOO-, -OCONH-, -NHCSO-and -OCSNH-, R representing an alkyl group.

According to another embodiment, at least one of the compounds X and Y is a polymer whose main chain is mainly formed from organosiloxane units.

According to another embodiment, at least one of the compounds X and Y is a polymer whose main chain is mainly formed from organosiloxane units.

Among the silicone compounds mentioned hereinbelow, some of them may have both film-forming properties and adhesive properties, depending, for example, on the silicone proportion thereof or on whether they are used as a mixture with a particular additive. It is consequently possible to modify the film-forming properties or the adhesive properties of such compositions according to the intended use, and this is in particular the case for "room-temperature-vulcanizable" reactive elastomeric silicones.

Compounds X and Y can react together at a temperature ranging between room temperature and 180°C. Advantageously, compounds X and Y are capable of reacting together at room temperature (20 ± 5°C) and atmospheric pressure, advantageously in the presence of a catalyst, via a hydrosilylation reaction or a condensation reaction, or a crosslinking reaction in the presence of a peroxide.

Compounds X and Y are capable of reacting via condensation, either in the presence of water (hydrolysis) by reaction of 2 compounds bearing alkoxysilane groups, or via "direct" condensation by reaction of a compound bearing alkoxysilane group(s) and a compound bearing silanol group(s) or by reaction of 2 compounds bearing silanol group(s).

When the condensation is performed in the presence of water, this water may in particular be water provided by an external source, for example premoistening of the hair (for example with a mister).

In this mode of reaction via condensation, compounds X and Y, which may be identical or different, may thus be chosen from silicone compounds whose main chain comprises at least two alkoxysilane groups and/or at least two silanol (Si-OH) groups, on the side and/or at the end of the chain.

In one advantageous embodiment, compounds X and/or Y are chosen from polyorganosiloxanes comprising at least two alkoxysilane groups. The term "alkoxysilane group" means a group comprising at least one -Si-OR portion, R being an alkyl group containing from 1 to 6 carbon atoms.

Compounds X and Y are especially chosen from polyorganosiloxanes comprising alkoxysilane end groups, more specifically those comprising at least 2 alkoxysilane end groups and preferably trialkoxysilane end groups.

Such polymers are described especially in documents US 3 175 993, US 4 772 675, US 4 871 827, US 4 888 380, US 4 898 910, US 4 906 719 and US 4 962 174, and WO 01/96450.

When the film-forming polymer according to the invention is dispersed in the organic solvent, the composition according to the invention advantageously comprises at least one stable dispersion of essentially spherical polymer particles of one or more polymers. Before incorporating them into the composition of the invention, the particles are generally dispersed in a physiologically acceptable liquid fatty phase, such as hydrocarbon-based oils or silicone oils. According to one embodiment, these dispersions are generally known as NADs (non-aqueous dispersions) of polymer, as opposed to networks, which are aqueous dispersions of polymer.

These dispersions may especially be in the form of polymer nanoparticles in stable dispersion in the said liquid organic phase. The nanoparticles preferably have a mean size of between 5 and 800 nm and better still between 50 and 500 nm. It is possible, however, to obtain polymer particle sizes ranging up to 1 µm.

The polymers in dispersion that may be used in the composition of the invention preferably have a molecular weight ranging from about 2000 to 10 000 000 and a Tg ranging from -100°C to 300°C and preferably from -10°C to 80°C.

Among the film-forming polymers in dispersion that may be mentioned are radical, acrylic or vinyl homopolymers or copolymers, preferably with a Tg of less than or equal to 40°C and especially ranging from -10°C to 30°C, used alone or as a mixture.

According to one embodiment, the polymer particles are stabilized with a stabilizer that is solid at room temperature, which may be a block polymer, a grafted polymer and/or a statistical polymer, alone or as a mixture. The stabilization may take place by any known means, and in particular by direct addition of the stabilizing polymer during the polymerization.

When an aqueous dispersion of polymer particles is used, the solids content of the said aqueous dispersion may be from about 3% to 60% and preferably from 10% to 50% by weight.

The size of the polymer particles in aqueous dispersion may be between 10 and 500 nm and is preferably between 20 and 150 nm, allowing the production of a film of noteworthy gloss. However, particle sizes ranging up to 1 micron may be used.

In a non-limiting manner, the preferred film-forming polymers are chosen from the following polymers or copolymers: polyurethanes, polyurethane-acrylics, polyureas, polyurea-polyurethanes, polyester-polyurethanes, polyether-polyurethanes, polyesters, polyester amides; acrylic and/or vinyl polymers or copolymers; acrylic-silicone copolymers; polyacrylamides; silicone polymers such as silicone polyurethane or acrylic polymers, fluoro polymers; celluloses; and mixtures thereof.

The hydrophobic film-forming polymers according to the invention may be selected on the basis of their mechanical properties. Such properties may be the flexibility, the hardness, the adhesion, the remanence, the resistance to water or to other chemical compounds, and the abrasion resistance. It is also possible to take advantage of the more versatile properties of block polymers (polymers constituted of two or more distinct polymer segments), grafted polymers (polymers containing a polymeric side chain grafted onto the homopolymer or copolymer backbone) or heteropolymers (polymers comprising two or more different monomers). In the copolymers, for example, the amount of hard and soft blocks has a significant impact on the properties of the polymer.

Furthermore, it is possible to mix two or more polymers in order to achieve the desired property. Examples of combinations may be polyurethane and polyacrylates, polyurethane and polyesters, two polymers having a silicone portion, or polyurethane and a polymer having a silicone portion.

According to one particular embodiment, the hydrophobic film-forming polymer is a nonionic polymer. According to another embodiment, the film-forming polymer is solid at 25°C, in the sense that no flowing is observed with the naked eye after one hour.

According to one particular embodiment, the hydrophobic film-forming polymer is a hybrid acrylic polymer. For the purposes of the present invention, a polymer synthesized from at least one compound (i) chosen from monomers bearing at least one (meth)acrylic acid group and/or esters of these acid monomers and/or amides of these acid monomers and from at least one compound (ii) different from the compounds (i), i.e. which does not comprises (meth)acrylic acid group and/or esters of these acid monomers and/or amides of these acid monomers, is intended for use.

The (meth)acrylic acid esters (also known as (meth)acrylates) are advantageously chosen from alkyl (meth)acrylates, in particular of C₁-C₃₀, preferably C₁-C₂₀ and better still C₁-C₁₀ alkyl, aryl (meth)acrylates, in particular of C₆-C₁₀ aryl, and hydroxyalkyl (meth)acrylates, in particular of C₂-C₆ hydroxyalkyl.

Alkyl (meth)acrylates that may be mentioned include methyl methacrylate, ethyl methacrylate, butyl methacrylate, isobutyl methacrylate, 2-ethylhexyl methacrylate, lauryl methacrylate and cyclohexyl methacrylate.

Hydroxyalkyl (meth)acrylates that may be mentioned include hydroxyethyl acrylate, 2-hydroxypropyl acrylate, hydroxyethyl methacrylate and 2-hydroxypropyl methacrylate.

Aryl (meth)acrylates that may be mentioned include benzyl acrylate and phenyl acrylate.

The (meth)acrylic acid esters that are particularly preferred are the alkyl (meth)acrylates.

According to the present invention, the alkyl group of the esters may be either fluorinated or perfluorinated, i.e. some or all of the hydrogen atoms of the alkyl group are substituted with fluorine atoms.

Examples of amides of the acid monomers that may be mentioned are (meth)acrylamides, and especially N-alkyl(meth)acrylamides, in particular of a C₂-C₁₂ alkyl. The N-alkyl(meth)acrylamides that may be mentioned include N-ethylacrylamide, N-t-butylacrylamide, N-t-octylacrylamide and N-undecylacrylamide.

As compounds (ii) different from the compounds (i), mention will be made, for example, of styrene monomers.

In particular, the acrylic polymer may be a styrene/acrylate copolymer, and especially a polymer chosen from copolymers derived from the polymerization of at least one styrene monomer and at least one C₁-C₂₀ and preferably C₁-C₁₀ alkyl acrylate monomer.

As styrene monomers that may be used in the invention, mention may be made of styrene and α-methylstyrene, and preferably styrene.

The C₁-C₁₀ alkyl acrylate monomer may be chosen from methyl acrylate, ethyl acrylate, propyl acrylate, butyl acrylate, hexyl acrylate, octyl acrylate and 2-ethylhexyl acrylate.

As acrylic polymers synthesized with a styrene compound, mention may be made of the styrene/acrylate copolymers sold under the name Joncryl 77 by the company BASF, under the name Yodosol GH41F by the company Akzo Nobel and under the name Syntran 5760 CG by the company Interpolymer.

As compound (ii), mention may also be made of compounds that interact via a process other than the radical polymerization of unsaturated compounds or the compounds derived from such a process. Such a process may be, for example, a polycondensation. As polycondensations, mention may be made of the formation of polyurethanes, polyesters or polyamides. Besides the acrylic monomer(s), the hybrid hydrophobic film-forming monomer of the invention will then contain the compound derived from the condensation process or the compounds that interact in the polycondensation process.

As film-forming hybrid acrylic copolymers of this type, mention may be made especially of the product sold under the reference Hybridur 875 Polymer Dispersion by the company Air Products & Chemicals.

As hybrid film-forming hydrophobic acrylic copolymer, use may also be made of the product sold under the reference Primal HG 1000 by the company Dow.

According to one particular embodiment, the hybrid film-forming acrylic polymer is a copolymer of (meth)acrylic acid ester(s) and of styrene.

According to one particular embodiment, the hydrophobic film-forming polymer is a silicone copolymer, preferably a linear block copolymer, that is to say an uncrosslinked copolymer, obtained by chain extension and not by crosslinking.

The term "block copolymer" (or "sequential copolymer") denotes a polymer comprising at least two distinct blocks (sequences). Each block of the polymer results from one type of monomer or from several types of different monomers. This means that each block can be composed of a homopolymer or of a copolymer, it being possible for this copolymer constituting the block to be in its turn a random or alternating copolymer.

The silicone copolymer used in the composition according to the invention preferably comprises at least two distinct silicone blocks, each block of the polymer resulting from one type of silicone monomer or from several types of different silicone monomers, such as mentioned below.

It should also be noted that the copolymer is "linear"; in other words, the structure of the polymer is neither branched nor star-branched nor grafted.

The linear block silicone copolymer is advantageously provided in the form of particles in dispersion in an aqueous medium.

The aqueous dispersion of block copolymer particles is a silicone-in-water (Sil/W) emulsion, the oily globules of which are composed of a silicone of high viscosity, so that these globules appear to form as "soft particles".

The size of the linear block silicone copolymer particles can vary widely. Preferably, in the present patent application, the linear block silicone copolymer particles generally exhibit a number-average size of less than or equal to 2 microns and preferably of less than or equal to 1 micron.

The aqueous dispersions of linear block silicone copolymer particles used in the composition according to the invention can be chosen in particular from those described in the document EP-A-874 017, the teaching of which is incorporated here by reference. According to this document, it is possible in particular to obtain the silicone copolymers constituting these particles by a chain extension reaction in the presence of a catalyst, starting from at least:
- (a) one polysiloxane (i) having at least one reactive group and preferably one or two reactive groups per molecule; and
- (b) one organosilicone compound (ii) which reacts with the polysiloxane (i) by a chain extension reaction.

In particular, the polysiloxane (i) is chosen from the compounds of formula (I): 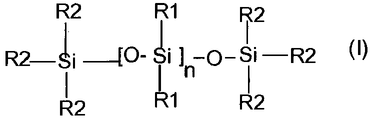 in which R₁ and R₂ represent, independently of one another, a hydrocarbon group having from 1 to 20 carbon atoms and preferably from 1 to 10 carbon atoms, such as methyl, ethyl, propyl or butyl, or an aryl group, such as phenyl, or a reactive group, and n is an integer greater than 1, provided that there are on average between one and two reactive groups per polymer.

The term "reactive group" is understood to mean any group capable of reacting with the organosilicone compound (ii) to form a block copolymer. Mention may be made, as reactive groups, of hydrogen; aliphatically unsaturated groups, and in particular vinyl, allyl or hexenyl groups; the hydroxyl group; alkoxy groups, such as methoxy, ethoxy or propoxy groups; alkoxy-alkoxy groups; the acetoxy group; amino groups, and mixtures thereof. Preferably, more than 90% and better still more than 98% of reactive groups are at the chain end, that is to say that the R₂ radicals generally constitute more than 90% and even 98% of the reactive groups.

n can in particular be an integer ranging from 2 to 100, preferably from 10 to 30 and better still from 15 to 25.

The polysiloxanes of formula (I) are linear polymers, that is to say comprising few branchings and generally less than 2 mol% of siloxane units. Furthermore, the R₁ and R₂ groups can optionally be substituted by amino groups, epoxy groups or sulfur-comprising, silicon-comprising or oxygen-comprising groups.

Preferably, at least 80% of the R₁ groups are alkyl groups and better still methyl groups.

Preferably, the reactive group R₂ at the chain end is an aliphatically unsaturated group and in particular a vinyl group.

Mention may in particular be made, as polysiloxanes (i), of dimethylvinylsiloxy-polydimethylsiloxane, a compound of formula (I) in which the R₁ radicals are methyl radicals and the R₂ radicals at the chain end are vinyl radicals while the other two R₂ radicals are methyl radicals.

The organosilicone compound (ii) can be chosen from polysiloxanes of formula (I) or compounds acting as chain-extending agent. If it is a compound of formula (I), the polysiloxane (i) will comprise a first reactive group and the organosilicone compound (ii) will comprise a second reactive group which will react with the first. If it is a chain-extending agent, it can be a silane, a siloxane (disiloxane or trisiloxane) or a silazane. Preferably, the organosilicone compound (ii) is a liquid organohydropolysiloxane of formula (II): 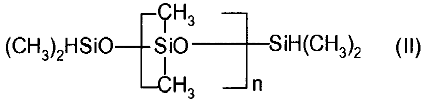 where n is an integer greater than 1 and preferably greater than 10, for example ranging from 2 to 100, preferably from 10 to 30 and better still from 15 to 25. According to a specific embodiment of the invention, n is equal to 20.

The silicone block copolymers used according to the invention are advantageously devoid of oxyalkylene group(s), in particular devoid of oxyethylene and/or oxypropylene group(s).

The catalyst of the reaction between the polysiloxane and the organosilicone compound can be chosen from metals and in particular from platinum, rhodium, tin, titanium, copper and lead. It is preferably platinum or rhodium.

The dispersion of silicone copolymer particles used in the composition according to the invention can in particular be obtained, for example, by mixing (a) water, (b) at least one emulsifier, (c) the polysiloxane (i), (d) the organosilicone compound (ii) and (e) a catalyst. Preferably, one of the constituents (c), (d) or (e) is added last to the mixture, in order for the chain-extending reaction to begin only in the dispersion.

Mention may be made, as emulsifiers capable of being used in the preparation process described above in order to obtain the aqueous dispersion of particles, of nonionic or ionic (anionic, cationic or amphoteric) emulsifiers. They are preferably nonionic emulsifiers which can be chosen from polyalkylene glycol ethers of fatty alcohol comprising from 8 to 30 carbon atoms and preferably from 10 to 22 carbon atoms; polyoxyalkylenated and in particular polyoxyethylenated sorbitan alkyl esters, where the alkyl radical comprises from 8 to 30 carbon atoms and preferably from 10 to 22 carbon atoms; polyoxyalkylenated and in particular polyoxyethylenated alkyl esters, where the alkyl radical comprises from 8 to 30 carbon atoms and preferably from 10 to 22 carbon atoms; polyethylene glycols; polypropylene glycols; diethylene glycols; and mixtures thereof. The amount of emulsifier(s) is generally from 1 % to 30% by weight, relative to the total weight of the reaction mixture.

The emulsifier used to obtain the aqueous dispersion of particles is preferably chosen from polyethylene glycol ethers of fatty alcohols and mixtures thereof and in particular polyethylene glycol ethers of alcohols comprising 12 or 13 carbon atoms and from 2 to 100 oxyethylene units and preferably from 3 to 50 oxyethylene units, and mixtures thereof. Mention may be made, for example, of C₁₂-C₁₃ Pareth-3, C₁₂-C₁₃ Pareth-23 and mixtures thereof.

According to a specific embodiment of the invention, the dispersion of silicone copolymer particles is obtained from dimethylvinylsiloxy-polydimethylsiloxane (or divinyldimethicone), as compound (i), and from the compound of formula (II) with preferably n=20, as compound (ii), preferably in the presence of a catalyst of platinum type, and the dispersion of particles is preferably obtained in the presence of C,₂-C,₃ Pareth-3 and C₁₂-C₁₃ Pareth-23, as emulsifiers.

Use may in particular be made, as dispersion of silicone copolymer particles, of the product sold under the name HMW 2220 by Dow Corning (CTFA name: divinyldimethicone/dimethicone copolymer/C₁₂-C₁₃ Pareth-3/C₁₂-C₁₃ Pareth-23), which is a 60% aqueous dispersion of divinyldimethicone/dimethicone copolymer comprising C₁₂-C₁₃ Pareth-3 and C₁₂-C₁₃ Pareth-23, said dispersion comprising approximately 60% by weight of copolymer, 2.8% by weight of C₁₂-C₁₃ Pareth-23, 2% by weight of C₁₂-C₁₃ Pareth-3 and 0.31% by weight of preservatives, the remainder to 100% being water.

The linear block silicone copolymer or copolymers can be present in an amount, as polymeric active materials, ranging from 0.1% to 30% by weight, better still from 0.5% to 20% by weight and even better still from 1% to 15% by weight, relative to the total weight of the composition.

According to one embodiment, the composition contains at least a hydrophobic film-forming polymer chosen from one hybrid hydrophobic film-forming acrylic polymer or polymers and linear block silicone copolymer or copolymers.

According to one embodiment, the composition contains as hydrophobic film-forming polymers at least one hybrid hydrophobic film-forming acrylic polymer or polymers and at least linear block silicone copolymer or copolymers. In this embodiment, the hybrid hydrophobic film-forming acrylic polymer or polymers and the linear block silicone copolymer or copolymers may be present in a weight ratio (as polymeric active materials) of hydrophobic film-forming acrylic polymer(s) to linear block silicone copolymer(s) ranging from 0.2 to 10, better still from 0.5 to 5 and even better still from 1 to 3.

The hydrophobic film-forming polymer(s) may be present in the composition in a content ranging from 0.1 % to 40% by weight, preferably ranging from 0.1 % to 30% by weight, preferably ranging from 0.5% to 20% by weight, preferentially ranging from 1% to 20% by weight and more preferentially ranging from 1% to 15% by weight relative to the total weight of the composition.

When the glass transition temperature of the hybrid hydrophobic film-forming acrylic polymer is too high for the desired use, for example a Tg of greater than 40°C, a plasticizer may be combined therewith so as to lower this temperature of the mixture used. The plasticizer may be chosen from the plasticizers usually used in the field of application, and especially from compounds that may be solvents for the polymer.

Preferably, the plasticizer has a molecular mass of less than or equal to 5000 g/mol, preferably less than or equal to 2000 g/mol, preferentially less than or equal to 1000 g/mol and even more preferentially less than or equal to 900 g/mol. The plasticizer advantageously has a molecular mass of greater than or equal to 100 g/mol.

Thus, the composition may also comprise at least one plasticizer. In particular, mention may be made, alone or as a mixture, of common plasticizers such as:
- glycols and derivatives thereof, such as diethylene glycol ethyl ether, diethylene glycol methyl ether, diethylene glycol butyl ether or diethylene glycol hexyl ether, ethylene glycol ethyl ether, ethylene glycol butyl ether or ethylene glycol hexyl ether;
- polyethylene glycols, polypropylene glycols, polyethylene glycol-polypropylene glycol copolymers, and mixtures thereof, especially high molecular weight polypropylene glycols, for example having a molecular mass ranging from 500 to 15 000, for instance
- glycol esters;
- propylene glycol derivatives and in particular propylene glycol phenyl ether, propylene glycol diacetate, dipropylene glycol ethyl ether, tripropylene glycol methyl ether and diethylene glycol methyl ether, dipropylene glycol butyl ether. Such compounds are sold by Dow Chemical under the names Dowanol PPH and Dowanol DPnB;
- acid esters, especially of carboxylic acids, such as citrates, phthalates, adipates, carbonates, tartrates, phosphates and sebacates;
- esters derived from the reaction of a monocarboxylic acid of formula R₁₁COOH with a diol of formula HOR₁₂OH in which R₁₁ and R₁₂, which may be identical or different, represent a linear, branched or cyclic, saturated or unsaturated hydrocarbon-based chain preferably containing from 3 to 15 carbon atoms, optionally comprising one or more heteroatoms such as N, O or S, in particular the monoesters resulting from the reaction of isobutyric acid and octanediol such as 2,2,4-trimethyl-1,3-pentanediol, such as the product sold under the reference Texanol Ester Alcohol by the company Eastman Chemical;
- oxyethylenated derivatives, such as oxyethylenated oils, especially plant oils, such as castor oil;
- mixtures thereof.

More particularly, the plasticizer may be chosen from esters of at least one carboxylic acid containing 1 to 7 carbon atoms and of a polyol comprising at least 4 hydroxyl groups.

The polyol may be a cyclized or uncyclized saccharide - polyhydroxyaldehyde (aldose) or polyhydroxyketone (ketose). The polyol is preferably a saccharide cyclized in hemiacetal form.

The polyol may be a monosaccharide or a polysaccharide comprising from 1 to 10 saccharides, preferably from 1 to 4 saccharides and more preferably one or two saccharides. The polyol may be chosen from erythritol, xylitol, sorbitol, glucose, sucrose, lactose and maltose.

The polyol is preferably a disaccharide. Among the disaccharides, mention may be made of sucrose (also known as α-D-glucopyranosyl-(1-2)-β-D-fructofuranose), lactose (also known as P-D-galactopyranosyl-(1-4)-(3-D-glucopyranose) and maltose (also known as α-D-glucopyranosyl-(1-4)-β-D-glucopyranose), and preferably sucrose.

The ester may consist of a polyol esterified with at least two different monocarboxylic acids, or with at least three different monocarboxylic acids.

The ester may be a copolymer of two esters, in particular a copolymer i) of a sucrose substituted with benzoyl groups and ii) of a sucrose substituted with acetyl and/or isobutyryl groups.

The carboxylic acid is preferably a monocarboxylic acid containing from 1 to 7 carbon atoms and preferably from 1 to 5 carbon atoms, chosen, for example, from acetic acid, n-propanoic acid, isopropanoic acid, n-butanoic acid, isobutanoic acid, tert-butanoic acid, n-pentanoic acid and benzoic acid.

The ester may be obtained from at least two different monocarboxylic acids. According to one embodiment, the acid is an unsubstituted linear or branched acid.

The acid is preferably chosen from acetic acid, isobutyric acid and benzoic acid, and mixtures thereof, and more preferentially.

According to one preferred embodiment, the ester is sucrose diacetate hexakis(2-methylpropanoate), such as the product sold under the name Sustane SAIB Food Grade Kosher by the company Eastman Chemical.

According to another embodiment, the plasticizer may be chosen from esters of an aliphatic or aromatic polycarboxylic acid and of an aliphatic or aromatic alcohol containing from 1 to 10 carbon atoms.

The aliphatic or aromatic alcohol comprises from 1 to 10 and preferably from 1 to 8 carbon atoms, for example from 1 to 6 carbon atoms. It may be chosen from the alcohols R1OH, such that R1 represents methyl, ethyl, propyl, isopropyl, butyl, hexyl, ethylhexyl, decyl, isodecyl, benzyl, or benzyl substituted with an alkyl comprising 1 to 3 carbon atoms, and mixtures thereof.

The aliphatic or aromatic polycarboxylic acid preferably contains from 3 to 12 carbon atoms, preferably from 3 to 10 carbon atoms and preferably from 3 to 8 carbon atoms, for example 6 or 8 carbon atoms.

The aliphatic or aromatic polycarboxylic acid is advantageously chosen from dicarboxylic acids and tricarboxylic acids.

Among the aliphatic dicarboxylic acids that may be mentioned are those of formula HOOC-(CH₂)ₙ-COOH, in which n is an integer ranging from 1 to 10 and preferably ranging from 2 to 8, for example equal to 2, 4, 6 or 8.

Dicarboxylic acids chosen from succinic acid, adipic acid and sebacic acid are preferred.

Among the aromatic dicarboxylic acids, mention may be made of phthalic acid.

Among the tricarboxylic acids, mention may be made of the triacids that correspond to formula 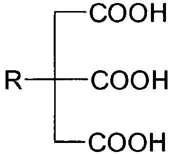 in which R represents a group -H, -OH or -OCOR' in which R' represents an alkyl group containing from 1 to 6 carbon atoms. Preferably, R represents a group -OCOCH₃.

The tricarboxylic acid is especially chosen from acetylcitric acid, butyroylcitric acid and citric acid.

Among the tricarboxylic acid esters that may be used are esters derived from citric acid (or citrates) such as tributyl acetyl citrate, triethyl acetyl citrate, triethylhexyl acetyl citrate, trihexyl acetyl citrate, trihexyl butyroyl citrate, triisodecyl citrate, triisopropyl citrate, tributyl citrate and tris(2-ethylhexyl) citrate. As commercial references of plasticizers mentioned above, mention may be made of the Citroflex range sold by Vertellus, especially, Citroflex A4 and Citroflex C2.

Among the adipic acid esters that may be mentioned are dibutyl adipate and bis(2-ethylhexyl) adipate.

Among the sebacic acid esters that may be mentioned are dibutyl sebacate, bis(2-ethylhexyl) sebacate, diethyl sebacate and diisopropyl sebacate.

Among the succinic acid esters that may be mentioned are bis(2-ethylhexyl) succinate and diethyl succinate.

Among the phthalic acid esters that may be mentioned are butyl benzyl phthalate, dibutyl phthalate, diethylhexyl phthalate, diethyl phthalate and dimethyl phthalate.

Advantageously, the plasticizer may be present in the composition in an amount such that the mass ratio between the hydrophobic film-forming polymer and the plasticizer is between 0.5 and 100, preferably between 1 and 50 and preferably between 1 and 10.

According to the invention, the composition applied to the hair contains at least one volatile solvent. In the context of the invention, the term "volatile solvent" means a compound that is liquid at room temperature (20°C) and at atmospheric pressure, having a vapour pressure at 20°C of greater than 0.1 mmHg, preferably between 0.1 and 300 mmHg and even more preferentially between 0.5 and 200 mmHg.

This volatile solvent may be water, a non-silicone organic solvent or a silicone organic solvent, or mixtures thereof. Volatile non-silicone organic solvents that may be mentioned include:
- volatile C₁-C₄ alkanols such as ethanol or isopropanol;
- volatile C₅-C₇ alkanes such as n-pentane, hexane, cyclopentane, 2,3-dimethylbutane, 2,2-dimethylbutane, 2-methylpentane or 3-methylpentane;
- esters of liquid C₁-C₂₀ acids and of volatile C₁-C₈ alcohols such as methyl acetate, n-butyl acetate, ethyl acetate, propyl acetate, isopentyl acetate or ethyl 3-ethoxypropionate;
- ketones that are liquid at room temperature and volatile, such as methyl ethyl ketone, methyl isobutyl ketone, diisobutyl ketone, isophorone, cyclohexanone or acetone;
- volatile polyols such as propylene glycol;
- volatile ethers such as dimethoxymethane, diethoxyethane or diethyl ether;
- volatile glycol ethers such as 2-butoxyethanol, butyl diglycol, diethylene glycol monomethyl ether, propylene glycol n-butyl ether or propylene glycol monomethyl ether acetate;
- volatile hydrocarbon-based oils such as volatile hydrocarbon-based oils containing from 8 to 16 carbon atoms, and mixtures thereof, and especially branched C₈-C₁₆ alkanes, for instance C₈-C₁₆ isoalkanes (also known as isoparaffins), isododecane, isodecane and, for example, the oils sold under the trade names Isopar or Permethyl, and mixtures thereof. Mention may also be made of isohexyl or isodecyl neopentanoate;
- volatile C₄-C₁₀ perfluoroalkanes such as dodecafluoropentane, tetradecafluorohexane or decafluoropentane;
- volatile perfluorocycloalkyls such as perfluoromethylcyclopentane, 1,3-perfluorodimethylcyclohexane and perfluorodecalin, sold, respectively, under the names Flutec PC1®, Flutec PC3® and Flutec PC6® by the company F2 Chemicals, and also perfluorodimethylcyclobutane and perfluoromorpholine;
- the volatile fluoroalkyl or heterofluoroalkyl compounds corresponding to the following formula:

   CH₃-(CH₂)ₙ-[Z]ₜX-CF₃

   in which t is 0 or 1; n is 0, 1, 2 or 3; X is a linear or branched divalent perfluoroalkyl radical containing from 2 to 5 carbon atoms, and Z represents O, S or NR, R being hydrogen or a radical -(CH₂)ₙ-CH₃ or -(CF₂)ₘ-CF₃, m being 2, 3, 4 or 5.

Among the volatile fluoroalkyl or heterofluoroalkyl compounds that may especially be mentioned are methoxynonafluorobutane sold under the names

MSX 4518® and HFE-7100® by the company 3M, and ethoxynonafluorobutane sold under the name HFE-7200® by the company 3M.

Preferably, the solvent is chosen such that its boiling point is less than 200°C.

According to one particular embodiment, the non-silicone organic solvent is chosen from ethanol, isopropanol, acetone and isododecane.

Volatile silicone solvents that may be mentioned include low-viscosity silicone compounds chosen from linear or cyclic silicones containing from 2 to 7 silicon atoms, these silicones optionally comprising alkyl or alkoxy groups containing from 1 to 10 carbon atoms, for example octamethylcyclotetrasiloxane, decamethylcyclopentasiloxane, dodecamethylcyclohexasiloxane, heptamethylhexyltrisiloxane, heptamethylethyltrisiloxane, heptamethyloctyltrisiloxane, octamethyltrisiloxane and decamethyltetrasiloxane, and mixtures thereof. According to one particular embodiment, the silicone compound is chosen from cyclopentadimethylsiloxane and dodecamethylcyclohexasiloxane.

According to one particular embodiment, the volatile silicone solvent has a viscosity of less than 50 centistokes.

Preferably, the volatile silicone is cyclic and chosen from decamethylcyclopentasiloxane, octamethyltrisiloxane and decamethyltetrasiloxane.

Examples include the decamethylcyclopentasiloxane sold under the name DC-245 by the company Dow Corning, the octamethyltrisiloxane sold under the name DC-200 Fluid 1 cst by the company Dow Corning, and the decamethyltetrasiloxane sold under the name DC-200 Fluid 1.5 cst by the company Dow Corning.

This cyclic volatile silicone generally has a low viscosity, for example a viscosity of less than 5 cSt at 25°C.

Preferably, the volatile silicone is cyclic and is the decamethylcyclopentasiloxane sold under the name DC-245 by the company Dow Corning.

The volatile solvent(s) may be present in the composition that is useful in the process of the invention in a content ranging from 0.1% to 95% by weight, preferably ranging from 1% to 70% by weight and preferentially ranging from 5% to 90% by weight relative to the total weight of the composition.

### Pigments

The composition may comprise pigments.

Such a composition makes it possible to obtain coloured and remanent coatings, without degradation of the keratin fibres, and with all the advantages described previously.

The term "pigments" means white or coloured particles of any shape, which are insoluble in the composition in which they are present.

The pigments that may be used are especially chosen from the organic and/or mineral pigments known in the art, especially those described in Kirk-Othmer's *Encyclopaedia of Chemical Technology* and in Ullmann's *Encyclopaedia of Industrial Chemistry.*

They may be natural, of natural origin, or non-natural.

These pigments may be in the form of powder or of pigmentary paste. They can be coated or uncoated.

The pigments may be chosen, for example, from mineral pigments, organic pigments, lakes, pigments with special effects such as nacres or glitter flakes, and mixtures thereof.

The pigment may be a mineral pigment. The term "mineral pigment" means any pigment that satisfies the definition in Ullmann's encyclopaedia in the chapter on inorganic pigments. Among the mineral pigments that are useful in the present invention, mention may be made of ochres such as red ochre (clay (in particular kaolinite) and iron hydroxide (for example haematite)), brown ochre (clay (in particular kaolinite) and limonite), yellow ochre (clay (in particular kaolinite) and goethite); titanium dioxide, optionally surface-treated; zirconium oxide or cerium oxide; zinc oxide, iron oxide (black, yellow or red) or chromium oxide; manganese violet, ultramarine blue, chromium hydrate and ferric blue; metal powders such as aluminium powder or copper powder.

Mention may also be made of carbonates of alkaline-earth metals (for example calcium or magnesium), silicon dioxide, quartz, and also any other compound used as inert filler in cosmetic compositions, provided that these compounds afford the composition colour or whiteness under the conditions in which they are used.

The pigment may be an organic pigment. The term "organic pigment" means any pigment that satisfies the definition in Ullmann's encyclopaedia in the chapter on organic pigments.

The organic pigment may especially be chosen from nitroso, nitro, azo, xanthene, pyrene, quinoline, anthraquinone, triphenylmethane, fluorane, phthalocyanin, metal complex, isoindolinone, isoindoline, quinacridone, perinone, perylene, diketopyrrolopyrrole, indigo, thioindigo, dioxazine, triphenylmethane and quinophthalone compounds.

Use may also be made of any mineral or organic compound that is insoluble in the composition and standard in cosmetics, provided that these compounds afford the composition colour or whiteness under the conditions in which they are used, for example guanine, which, according to the refractive index composition, is a pigment.

In particular, the white or coloured organic pigments may be selected from carmine lake, carbon black, aniline black, azo yellow, quinacridone, phthalocyanine blue, the blue pigments codified in the Color Index under the references Cl 42090, 69800, 69825, 73000, 74100 and 74160, the yellow pigments codified in the Color Index under the references Cl 11680, 11710, 15985, 19140, 20040, 21100, 21108, 47000 and 47005, the green pigments codified in the Color Index under the references Cl 61565, 61570 and 74260, the orange pigments codified in the Color Index under the references Cl 11725, 15510, 45370 and 71105, the red pigments codified in the Color Index under the references Cl 12085, 12120, 12370, 12420, 12490, 14700, 15525, 15580, 15620, 15630, 15800, 15850, 15865, 15880, 17200, 26100, 45380, 45410, 58000, 73360, 73915 and 75470, and the pigments obtained by oxidative polymerization of indole or phenolic derivatives as described in patent FR 2 679 771.

Examples that may also be mentioned include pigmentary pastes of organic pigments, such as the products sold by the company Hoechst under the names:
- Cosmenyl Yellow IOG: Pigment Yellow 3 (CI 11710);
- Cosmenyl Yellow G: Pigment Yellow 1 (CI 11680);
- Cosmenyl Orange GR: Pigment Orange 43 (CI 71105);
- Cosmenyl Red R: Pigment Red 4 (CI 12085);
- Cosmenyl Carmine FB: Pigment Red 5 (CI 12490);
- Cosmenyl Violet RL: Pigment Violet 23 (CI 51319);
- Cosmenyl Blue A2R: Pigment Blue 15.1 (CI 74160);
- Cosmenyl Green GG: Pigment Green 7 (CI 74260);
- Cosmenyl Black R: Pigment Black 7 (CI 77266).

The pigments in accordance with the invention may also be in the form of composite pigments, as described in patent EP 1 184 426. These composite pigments may be especially composed of particles comprising a mineral core, at least one binder for binding the organic pigments to the core, and at least one organic pigment at least partially covering the core.

The organic pigment may also be a lake. The term "lake" means dyes adsorbed onto insoluble particles, the assembly thus obtained remaining insoluble during use.

The inorganic substrates onto which the dyes are adsorbed are, for example, alumina, silica, calcium sodium borosilicate or calcium aluminium borosilicate, and aluminium.

Among the dyes, mention may be made of carminic acid. Mention may also be made of the dyes known under the following names: D&C Red 21 (CI 45 380), D&C Orange 5 (CI 45 370), D&C Red 27 (CI 45 410), D&C Orange 10 (CI 45 425), D&C Red 3 (CI 45 430), D&C Red 4 (CI 15 510), D&C Red 33 (CI 17 200), D&C Yellow 5 (CI 19 140), D&C Yellow 6 (CI 15 985), D&C Green (CI 61 570), D&C Yellow 10 (CI 47 005), D&C Green 3 (CI 42 053), D&C Blue 1 (CI 42 090).

An example of a lake that may be mentioned is the product known under the following name: D&C Red 7 (CI 15 850:1).

The pigment may also be a pigment with special effects. The term "pigments with special effects" means pigments that generally create a coloured appearance (characterized by a certain shade, a certain vivacity and a certain level of luminance) that is non-uniform and that changes as a function of the conditions of observation (light, temperature, angles of observation, etc.). They thus contrast with coloured pigments that afford a conventional uniform opaque, semi-transparent or transparent shade. Several types of pigment with special effects exist: those with a low refractive index, such as fluorescent, photochromic or thermochromic pigments, and those with a high refractive index, such as nacres, interference pigments or glitter flakes.

Examples of pigments with special effects that may be mentioned include nacreous pigments such as mica coated with titanium or with bismuth oxychloride, coloured nacreous pigments such as titanium mica with iron oxides, titanium mica especially with ferric blue or with chromium oxide, titanium mica with an organic pigment of the abovementioned type, and also nacreous pigments based on bismuth oxychloride. Nacreous pigments that may be mentioned include the nacres Cellini sold by Engelhard (mica-TiO₂-lake), Prestige sold by Eckart (mica-TiO₂), Prestige Bronze sold by Eckart (mica-Fe₂O₃) and Colorona sold by Merck (mica-TiO₂-Fe₂O₃).

Mention may be made of the gold-coloured nacres sold especially by the company Engelhard under the name Brilliant gold 212G (Timica), Gold 222C (Cloisonne), Sparkle gold (Timica), Gold 4504 (Chromalite) and Monarch gold 233X (Cloisonne); the bronze nacres sold especially by the company Merck under the name Bronze fine (17384) (Colorona) and Bronze (17353) (Colorona) and by the company Engelhard under the name Super bronze (Cloisonne); the orange nacres sold especially by the company Engelhard under the name Orange 363C (Cloisonne) and Orange MCR 101 (Cosmica) and by the company Merck under the name Passion orange (Colorona) and Matte orange (17449) (Microna); the brown nacres sold especially by the company Engelhard under the name Nu-antique copper 340XB (Cloisonne) and Brown CL4509 (Chromalite); the nacres with a copper tint sold especially by the company Engelhard under the name Copper 340A (Timica); the nacres with a red tint sold especially by the company Merck under the name Sienna fine (17386) (Colorona); the nacres with a yellow tint sold especially by the company Engelhard under the name Yellow (4502) (Chromalite); the red nacres with a gold tint sold especially by the company Engelhard under the name Sunstone G012 (Gemtone); the pink nacres sold especially by the company Engelhard under the name Tan opale G005 (Gemtone); the black nacres with a gold tint sold especially by the company Engelhard under the name Nu antique bronze 240 AB (Timica), the blue nacres sold especially by the company Merck under the name Matte blue (17433) (Microna), the white nacres with a silvery tint sold especially by the company Merck under the name Xirona Silver, and the golden-green pink-orange nacres sold especially by the company Merck under the name Indian summer (Xirona), and mixtures thereof.

Still as examples of nacres, mention may also be made of particles comprising a borosilicate substrate coated with titanium oxide.

Particles having a glass substrate coated with titanium oxide are especially sold under the name Metashine MC1080RY by the company Toyal.

Finally, examples of nacres that may also be mentioned include polyethylene terephthalate flakes, especially those sold by the company Meadowbrook Inventions under the name Silver 1 P 0.004X0.004 (silver flakes).

Multilayer pigments based on synthetic substrates such as alumina, silica, sodium calcium borosilicate or calcium aluminium borosilicate, and aluminium, may also be envisaged.

The particles with special effects may also be chosen from reflective particles, i.e. especially from particles whose size, structure, especially the thickness of the layer(s) of which they are made and their physical and chemical nature, and surface state, allow them to reflect incident light. This reflection may, where appropriate, have an intensity sufficient to create at the surface of the composition or of the mixture, when it is applied to the support to be made up, points of overbrightness that are visible to the naked eye, i.e. more luminous points that contrast with their environment by appearing to sparkle.

The reflective particles may be selected so as not to significantly alter the colouration effect generated by the colouring agents with which they are combined, and more particularly so as to optimize this effect in terms of colour yield. They may more particularly have a yellow, pink, red, bronze, orange, brown, gold and/or coppery colour or tint.

These particles may have varied forms and may especially be in platelet or globular form, in particular in spherical form.

The reflective particles, whatever their form, may or may not have a multilayer structure and, in the case of a multilayer structure, may have, for example, at least one layer of uniform thickness, in particular of a reflective material.

When the reflective particles do not have a multilayer structure, they may be composed, for example, of metal oxides, especially titanium or iron oxides obtained synthetically.

When the reflective particles have a multilayer structure, they may comprise, for example, a natural or synthetic substrate, especially a synthetic substrate at least partially coated with at least one layer of a reflective material, especially of at least one metal or metallic material. The substrate may be made of one or more organic and/or inorganic materials.

More particularly, it may be chosen from glasses, ceramics, graphite, metal oxides, aluminas, silicas, silicates, especially aluminosilicates and borosilicates, and synthetic mica, and mixtures thereof, this list not being limiting.

The reflective material may comprise a layer of metal or of a metallic material.

Reflective particles are described especially in documents JP-A-09188830, JP-A-10158450, JP-A-10158541, JP-A-07258460 and JP-A-05017710.

Again as an example of reflective particles comprising a mineral substrate coated with a layer of metal, mention may also be made of particles comprising a silver-coated borosilicate substrate.

Particles with a silver-coated glass substrate, in the form of platelets, are sold under the name Microglass Metashine REFSX 2025 PS by the company Toyal. Particles with a glass substrate coated with nickel/chromium/molybdenum alloy are sold under the name Crystal Star GF 550 and GF 2525 by this same company.

Use may also be made of particles comprising a metallic substrate such as silver, aluminium, iron, chromium, nickel, molybdenum, gold, copper, zinc, tin, manganese, steel, bronze or titanium, said substrate being coated with at least one layer of at least one metal oxide such as titanium oxide, aluminium oxide, iron oxide, cerium oxide, chromium oxide or silicon oxides, and mixtures thereof.

Examples that may be mentioned include aluminium powder, bronze powder or copper powder coated with Si0₂ sold under the name Visionaire by the company Eckart.

Mention may also be made of pigments with an interference effect not bound to a substrate, for instance liquid crystals (Helicones HC from Wacker), holographic interference flakes (Geometric Pigments or Spectra f/x from Spectratek). Special effect pigments also comprise fluorescent pigments, whether substances which are fluorescent in daylight or which produce ultraviolet fluorescence, phosphorescent pigments, photochromic pigments, thermochromic pigments and quantum dots, for example sold by Quantum Dots Corporation.

Quantum dots are luminescent semiconductor nanoparticles capable of emitting, under light excitation, radiation exhibiting a wavelength of between 400 nm and 700 nm. These nanoparticles are known from the literature. In particular, they may be manufactured according to the processes described, for example, in US 6 225 198 or US 5 990 479, in the publications cited therein and also in the following publications: Dabboussi B.O. et al. "(CdSe)ZnS core-shell quantum dots: synthesis and characterization of a size series of highly luminescent nanocristallites" Journal of Physical Chemistry B, vol. 101, 1997, pp. 9463-9475 and Peng, Xiaogang et al., "Epitaxial Growth of highly Luminescent CdSe/CdS core/shell nanocrystals with photostability and electronic accessibility" Journal of the American Chemical Society, vol. 119, No. 30, pp. 7019-7029.

The variety of pigments that may be used in the present invention makes it possible to obtain a wide range of colours, and also particular optical effects such as metallic effects or interference effects.

The size of the pigment used in the cosmetic composition according to the present invention is generally between 10 nm and 200 µm, preferably between 20 nm and 80 µm and even more preferably between 30 nm and 50 µm.

The pigments may be dispersed in the product by means of a dispersant.

The dispersant serves to protect the dispersed particles against agglomeration or flocculation. This dispersant may be a surfactant, an oligomer, a polymer or a mixture of several thereof, bearing one or more functionalities with strong affinity for the surface of the particles to be dispersed. In particular, they can physically or chemically attach to the surface of the pigments. These dispersants also contain at least one functional group that is compatible with or soluble in the continuous medium. In particular, 12-hydroxystearic acid esters and C₈ to C₂₀ fatty acid esters of polyols such as glycerol or diglycerol are used, such as poly(12-hydroxystearic acid) stearate with a molecular weight of about 750 g/mol, such as the product sold under the name Solsperse 21 000 by the company Avecia, polyglyceryl-2 dipolyhydroxystearate (CTFA name) sold under the reference Dehymyls PGPH by the company Henkel, or polyhydroxystearic acid such as the product sold under the reference Arlacel P100 by the company Uniqema, and mixtures thereof.

As other dispersants that may be used in the compositions of the invention, mention may be made of quaternary ammonium derivatives of polycondensed fatty acids, for instance Solsperse 17 000 sold by the company Avecia, and polydimethylsiloxane/oxypropylene mixtures such as those sold by the company Dow Corning under the references DC2-5185 and DC2-5225 C.

The pigments used in the cosmetic composition according to the invention may be surface-treated with an organic agent.

Thus, the pigments that have been surface-treated beforehand, which are useful in the context of the invention, are pigments that have totally or partially undergone a surface treatment of chemical, electronic, electrochemical, mechanochemical or mechanical nature, with an organic agent such as those described especially in Cosmetics and Toiletries, February 1990, vol. 105, pp. 53-64, before being dispersed in the composition that is useful in the invention. These organic agents may be chosen, for example, from waxes, for example carnauba wax and beeswax; fatty acids, fatty alcohols and derivatives thereof, such as stearic acid, hydroxystearic acid, stearyl alcohol, hydroxystearyl alcohol and lauric acid and derivatives thereof; anionic surfactants; lecithins; sodium, potassium, magnesium, iron, titanium, zinc or aluminium salts of fatty acids, for example aluminium stearate or laurate; metal alkoxides; polyethylene; (meth)acrylic polymers, for example polymethyl methacrylates; polymers and copolymers containing acrylate units; alkanolamines; silicone compounds, example silicones, polydimethylsiloxanes; organofluorine compounds, for example perfluoroalkyl ethers; fluorosilicone compounds.

The surface-treated pigments that are useful in the cosmetic composition according to the invention may also have been treated with a mixture of these compounds and/or may have undergone several surface treatments.

The surface-treated pigments that are useful in the context of the present invention may be prepared according to surface-treatment techniques that are well known to those skilled in the art, or may be commercially available in the required form.

Preferably, the surface-treated pigments are coated with an organic layer.

The organic agent with which the pigments are treated may be deposited on the pigments by evaporation of solvent, chemical reaction between the molecules of the surface agent or creation of a covalent bond between the surface agent and the pigments.

The surface treatment may thus be performed, for example, by chemical reaction of a surface agent with the surface of the pigments and creation of a covalent bond between the surface agent and the pigments or the fillers. This method is especially described in patent US 4 578 266.

An organic agent covalently bonded to the pigments will preferably be used.

The agent for the surface treatment may represent from 0.1 % to 50% by weight, preferably from 0.5% to 30% by weight and even more preferentially from 1% to 10% by weight relative to the total weight of the surface-treated pigments.

Preferably, the surface treatments of the pigments are chosen from the following treatments:
- a PEG-silicone treatment, for instance the AQ surface treatment sold by LCW;
- a methicone treatment, for instance the SI surface treatment sold by LCW;
- a dimethicone treatment, for instance the Covasil 3.05 surface treatment sold by LCW;
- a dimethicone/trimethyl siloxysilicate treatment, for instance the Covasil 4.05 surface treatment sold by LCW;
- an aluminium dimyristate treatment, for instance the MI surface treatment sold by Miyoshi;
- a perfluoropolymethylisopropyl ether treatment, for instance the FHC surface treatment sold by LCW;
- an isostearyl sebacate treatment, for instance the HS surface treatment sold by Miyoshi;
- a disodium stearoyl glutamate treatment, for instance the NAI surface treatment sold by Miyoshi;
- a perfluoroalkyl phosphate treatment, for instance the PF surface treatment sold by Daito;
- an acrylate/dimethicone copolymer and perfluoroalkyl phosphate treatment, for instance the FSA treatment sold by Daito;
- a polymethylhydrogenosiloxane/perfluoroalkyl phosphate treatment, for instance the FS01 surface treatment sold by Daito;
- an acrylate/dimethicone copolymer treatment, for instance the ASC surface treatment sold by Daito;
- an isopropyl titanium triisostearate treatment, for instance the ITT surface treatment sold by Daito;
- an acrylate copolymer treatment, for instance the APD surface treatment sold by Daito;
- a perfluoroalkyl phosphate/isopropyl titanium triisostearate treatment, for instance the PF + ITT surface treatment sold by Daito.

Preferably, the pigment is chosen from mineral or mixed mineral-organic pigments.

The amount of pigment(s) may range from 0.01% to 30%, more particularly from 0.05% to 20% and preferably from 0.1 % to 15% by weight relative to the total weight of the composition.

The composition of the invention may also contain other non-volatile organic solvents, such as:
- non-volatile aromatic alcohols such as benzyl alcohol or phenoxyethanol;
- non-volatile esters of liquid C₁-C₂₀ acids and of C₁-C₈ alcohols, such as isopropyl myristate;
- ethylene carbonate, propylene carbonate or butylene carbonate;
- non-volatile polyols such as glycerol, ethylene glycol, dipropylene glycol or butylene glycol;
- non-volatile glycol ethers, for instance diethylene glycol monomethyl ether or dipropylene glycol mono-n-butyl ether;
- non-volatile hydrocarbon-based oils such as isohexadecane;
- non-volatile liquid C₁₀-C₃₀ fatty alcohols such as oleyl alcohol; esters of liquid C₁₀-C₃₀ fatty alcohols such as benzoates of C₁₀-C₃₀ fatty alcohols and mixtures thereof; polybutene oil, isononyl isononanoate, isostearyl malate, pentaerythrityl tetraisostearate or tridecyl trimellitate;
- non-volatile perfluoro solvents such as perfluoroperhydrophenanthrene, sold under the name Flutec PC11® by the company F2 Chemicals.

When the composition comprises pigments. Such a composition makes it possible to obtain coloured and remanent coatings, without degradation of the keratin fibres.

### Thickener

The composition that is useful in the device or the process of the invention may comprise at least one thickener. This thickener may be chosen from mineral or organic, polymeric or non-polymeric thickeners, and mixtures thereof.

The term "thickener" means a compound that modifies the rheology of the medium into which it is incorporated.

According to one particular embodiment of the invention, the composition comprises at least one mineral thickener.

Preferably, the thickener(s) are chosen from fumed silica and clays, or mixtures thereof.

The fumed silicas may be obtained by high-temperature pyrolysis of a volatile silicon compound in an oxhydric flame, producing a finely divided silica. This process makes it possible in particular to obtain hydrophilic silicas which exhibit a large number of silanol groups at their surface. Such hydrophilic silicas are sold, for example, under the names Aerosil 130®, Aerosil 200®, Aerosil 255®, Aerosil 300® and Aerosil 380® by the company Degussa, and Cab-O-Sil HS-5®, Cab-O-Sil EH-5®, Cab-O-Sil LM-130®, Cab-O-Sil MS-55® and Cab-O-Sil M-5® by the company Cabot.

It is possible to chemically modify the surface of the said silica, via a chemical reaction generating a reduction in the number of silanol groups. It is possible in particular to replace silanol groups with hydrophobic groups: a hydrophobic silica is then obtained.

The hydrophobic groups can be:
- trimethylsiloxyl groups, which are obtained in particular by treating fumed silica in the presence of hexamethyldisilazane. Silicas thus treated are known as "Silica silylate" according to the CTFA (6th edition, 1995). They are sold, for example, under the references Aerosil R812® by the company Degussa and Cab-O-Sil TS-530® by the company Cabot.
- dimethylsilyloxyl or polydimethylsiloxane groups, which are obtained in particular by treating fumed silica in the presence of polydimethylsiloxane or dimethyldichlorosilane. Silicas thus treated are known as "Silica dimethyl silylate" according to the CTFA (6th edition, 1995). They are sold, for example, under the references Aerosil R972® and Aerosil R974® by the company Degussa, and Cab-O-Sil TS-610® and Cab-O-Sil TS-720® by the company Cabot.

The fumed silica preferably exhibits a particle size which can be nanometric to micrometric, for example ranging from approximately 5 to 200 nm.

Clays are products that are already well known per se, which are described, for. example, in the publication Minéralogie des argiles [Mineralogy of Clays], S. Caillère, S. Hénin, M. Rautureau, 2nd Edition 1982, Masson.

Clays are silicates containing a cation that may be chosen from calcium, magnesium, aluminium, sodium, potassium and lithium cations, and mixtures thereof.

Examples of such products that may be mentioned include clays of the smectite family such as montmorillonites, hectorites, bentonites, beidellites and saponites, and also of the vermiculite, stevensite and chlorite families.

These clays may be of natural or synthetic origin. Clays that are cosmetically compatible and acceptable with keratin materials are preferably used.

As clays that may be used according to the invention, mention may be made of synthetic hectorites (also known as laponites), for instance the products sold by the company Laporte under the name Laponite XLG, Laponite RD and Laponite RDS (these products are sodium magnesium silicates and in particular sodium lithium magnesium silicates); bentonites, for instance the product sold under the name Bentone HC by the company Rheox; magnesium aluminium silicates, especially hydrated, for instance the product sold by the Vanderbilt Company under the name Veegum Ultra, or calcium silicates and especially the product in synthetic form sold by the company CELITE ET WALSH ASS under the name Micro-cel C.

The organophilic clay may be chosen from montmorillonite, bentonite, hectorite, attapulgite and sepiolite, and mixtures thereof. The clay is preferably a bentonite or a hectorite.

These clays can be modified with a chemical compound chosen from quaternary ammoniums, tertiary amines, amine acetates, imidazolines, amine soaps, fatty sulfates, alkylarylsulfonates, amine oxides and their mixtures.

Mention may be made, as organophilic clays, of quaternium-18 bentonites, such as those sold under the names Bentone 3, Bentone 38 and Bentone 38V by Rheox, Tixogel VP by United Catalyst and Claytone 34, Claytone 40 and Claytone XL by Southern Clay; stearalkonium bentonites, such as those sold under the names Bentone 27 by Rheox, Tixogel LG by United Catalyst and Claytone AF and Claytone APA by Southern Clay; and quaternium-18/benzalkonium bentonites, such as those sold under the names Claytone HT and Claytone PS by Southern Clay.

The thickener may also be chosen from organic compounds.

Examples that may be mentioned include the following polymeric or non-polymeric products:
- C₁₀-C₃₀ fatty amides such as lauric acid diethanolamide,
- the polyglyceryl (meth)acrylate polymers sold under the names Hispagel and Lubragel by the companies Hispano Quimica or Guardian,
- polyvinylpyrrolidone,
- polyvinyl alcohol,
- crosslinked acrylamide polymers and copolymers, such as those sold under the names PAS 5161 or Bozepol C by the company Hoechst, Sepigel 305 by the company SEPPIC, or
- the crosslinked methacryloyloxyethyltrimethylammonium chloride homopolymers sold under the name Salcare SC95 by the company Allied Colloid,
- associative polymers and especially associative polyurethanes.

Such thickeners are especially described in patent application EP-A-1 400 234.

Mention may also be made of the following thickeners, in particular if the compositions contain oily compounds:
- organophilic clays;
- hydrophobic fumed silicas.

More precisely, organophilic clays are clays modified with chemical compounds that make the clay able to swell.

Preferably, the composition comprises at least one mineral thickener, which is preferably chosen from clays and even more advantageously from smectites, preferably bentonites.

According to one preferred embodiment, the composition that is useful in the invention comprises at least one thickener. This or these thickener(s) may then be present in a total content ranging from 0.1 % to 10% by weight relative to the weight of the composition.

The composition according to the invention comprises water, which may preferably be present in a content ranging from 20% to 98% by weight relative to the weight of the composition.

According to one particular embodiment, the composition that is useful in the device or the process of the invention contains an odorous compound or a mixture of odorous compounds, such as a perfume. Odorous compounds and perfumes that may be mentioned include those described in the article Perfumes by William L. Schreiber, pp. 171-201 volume 18 of the 4th edition of Kirk Othmer's Encyclopaedia of Chemical Technology, 1996.

The compositions may also contain at least one agent usually used in cosmetics other than the compounds already mentioned, chosen, for example, from reducing agents, fatty substances, plant oils, softeners, antifoams, moisturizers, UV-screening agents, peptizers, solubilizers, anionic, cationic, nonionic or amphoteric surfactants, proteins, vitamins, propellants, oxyethylenated or non-oxyethylenated waxes, and C₁₀-C₃₀ fatty acids such as stearic acid or lauric acid.

The above additives are generally present in an amount for each of them of between 0.01 % and 20% by weight relative to the weight of the composition.

Needless to say, a person skilled in the art will take care to select this or these optional additive(s) such that the advantageous properties intrinsically associated with the formation of the coating in accordance with the invention are not, or are not substantially, adversely affected.

The composition according to the invention may especially be in the form of a suspension, a dispersion, a gel, an emulsion, especially an oil-in-water (O/W) or water-in-oil (WIO) emulsion, or a multiple emulsion (W/O/W or polyol/0/W or O/W/O), in the form of a cream, a mousse, a stick, a dispersion of vesicles, especially of ionic or nonionic lipids, a two-phase or multi-phase lotion, a spray or a paste. The composition may also be in the form of a lacquer.

A person skilled in the art may select the appropriate galenical form, and also the method for preparing it, on the basis of his general knowledge, taking into account firstly the nature of the constituents used, especially their solubility in the support, and secondly the intended use of the composition.

The composition described above may be used on wet or dry keratin fibres, and also on any type of fair or dark, natural or dyed, permanent-waved, bleached or relaxed fibres.

According to one particular embodiment of the process of the invention, the fibres are washed before application of the composition described above.

After application of the composition, the fibres may be left to dry or dried, for example at a temperature above or equal to 30°C. The drying, if it is performed, may be performed immediately after the application or after a leave-on time that may range from 1 minute to 30 minutes.

Preferably, if the fibres are dried, then in addition to supplying heat, they are dried with a flow of air. This flow of air during drying makes it possible to improve the individualization of the coating.

During drying, a mechanical action may be exerted on the locks, such as combing, brushing or running the fingers through the hair. This operation may similarly be performed once the fibres have been dried, naturally or otherwise.

The drying step of the process of the invention may be performed with a hood, a hairdryer, a smoothing iron, a Climazon, etc.

When the drying step is performed with a hood or a hairdryer, the drying temperature is between 40 and 110°C and preferably between 50 and 90°C.

After drying, the head of hair may be shaped by the fingers or by using a device such as a comb, a brush, straightening tongs or a crimping iron. After such a treatment of the fibres, the shape given is shampoo-fast.

When the drying step is performed with a straightening or crimping iron, the drying temperature is between 110 and 220°C and preferably between 140 and 200°C.

When the composition that is useful in the invention contains one or more pigments, the keratin fibre treatment process is then a process for dyeing keratin fibres.

The invention will be illustrated more fully with the aid of the non-limiting examples that follow. Unless otherwise mentioned, the amounts indicated are expressed in grams.

### EXAMPLES

### Composition examples:

| Composition A | |
|---|---|
| BioPSA DC 7-4405 at 40% in isododecane, sold by Dow Corning | 17.5 g |
| α,ω-Dihydroxylated PDMS gum of high molecular weight | 1.5 g |
| Nacre of mica coated with brown iron oxide, sold by Eckart under the name Prestige Soft Bronze | 7 g |
| Isododecane | qs100g |

Composition A is placed on a lock with a tone depth of 4 using the applicator described previously. The lock is then dried with a hairdryer at a temperature of 80°C for 2 minutes. A lock dyed brown whose hairs are individualized and whose colour is homogeneous and shampoo-fast to several shampoo washes is obtained. The lock feels soft and close to a natural feel.

| Composition B | |
|---|---|
| Styrene/acrylate polymer in aqueous dispersion, sold by BASF under the name Joncryl 77 | 21 g, i.e. 10% AM |
| Clay (magnesium aluminium silicate) sold by the company Vanderbilt under the name Veegum Granules | 2 g |
| Black 2 as an aqueous dispersion, from Daito Kasei Kogyo under the name WD-CB2 | 1.8 g, i.e. 0.45% AM |
| Water | qs 100 g |

Composition B is placed on a lock of grey hair using the particular applicator described previously. After a few seconds at room temperature, the lock of hair is dried and the hair is coloured black, the colour is homogeneous and shampoo-fast to several shampoo washes. The hairs are individualized with the fingers or using a comb and/or a brush. The lock feels soft and close to a natural feel.

| Composition C | |
|---|---|
| Styrene/acrylate polymer in aqueous dispersion, sold by BASF under the name Joncryl 77 | 21.2 g, i.e. 10% AM |
| Divinyl dimethicone/dimethicone copolymer as an aqueous emulsion, sold by Dow Corning under the reference HMW 2220 Nonionic Emulsion | 8.3 g, i.e. 5% AM |
| *Trésor* perfume from Lancôme | 0.5 g |
| Water | qs 100 g |

Composition C is placed on a lock of hair using the particular applicator. After a few seconds at room temperature, the lock of hair is dried and perfumed. The hair remains perfumed at least until the first shampoo wash. The hairs are individualized with the fingers or using a comb and/or a brush. The lock feels soft and close to a natural feel.

| Composition D | |
|---|---|
| Styrene/acrylate polymer in aqueous dispersion, sold by BASF under the name Joncryl 77 | 21.2 g, i.e. 10% AM |
| Divinyl dimethicone/dimethicone copolymer as an aqueous emulsion, sold by Dow Corning under the reference HMW 2220 Nonionic Emulsion | 8.3 g, i.e. 5% AM |
| Clay (magnesium aluminium silicate) sold by the company Vanderbilt under the name Veegum Granules | 2 g |
| Water | qs 100 g |

Composition D is placed on a lock of fine hair using the particular applicator. After a few seconds at room temperature, the hair is dry and has an added feel of body. The treatment is remanent to several shampoo washes. The hairs are individualized with the fingers or using a comb and/or a brush. The lock feels soft and close to a natural feel.

## Claims

1. Device (1) for applying a keratin fibre treatment composition, comprising:
v) an applicator means (2) that is capable of retaining an amount of the treatment composition (P) in a container (20),
vi) a holding member (4) that can engage with the applicator means (2), in order, during a longitudinal movement of the device (1) relative to a lock of keratin fibres, to hold the said lock in engagement with the applicator means (2) so as to enable the lock to be coated with the treatment composition (P),
vii) the said applicator means (2) comprises an applicator tip (30) mounted on the container (20) and comprising an outlet orifice (31) equipped with an opening/closing member (32), which, in a first position, closes off the said outlet orifice, and which, in a second position, at least partially releases the said outlet orifice, the passage from the first to the second position taking place in response to a stress exerted on the opening/closing member (32) by engaging the said lock between the holding member and this opening/closing member (32);
viii)the treatment composition (P) comprising at least one hydrophobic film-forming polymer and at least one volatile solvent.

2. Device according to the preceding claim, **characterized in that** the holding member (4) is mobile relative to the applicator means (2).

3. Device according to either of the preceding claims, **characterized in that** the holding member (4) and the applicator means (2) form two separate pieces or alternatively are linked via a connecting means (50) and form a single piece.

4. Device according to any one of the preceding claims, **characterized in that** the applicator tip (3) comprises a surface (37) that is capable of storing and releasing the treatment composition (P).

5. Device according to any one of the preceding claims, in which the hydrophobic film-forming polymer is chosen from polymers or copolymers based on polyurethane, polyacrylate, vinyl ester copolymers, silicone resins, polyurea/polyurethane silicones, copolymers based on silicone resin and on dimethiconol, and reactive silicones, preferably hybrid acrylate polymers.

6. Device according to any one of the preceding claims, in which the volatile solvent is water or an organic solvent chosen from volatile C₁-C₄ alkanols, volatile C₅-C₇ alkanes, volatile hydrocarbon-based oils containing from 8 to 16 carbon atoms, and especially branched C₈-C₁₆ alkanes, volatile silicone solvents including low-viscosity silicone compounds chosen from linear or cyclic silicones containing from 2 to 7 silicon atoms, these silicones optionally comprising alkyl or alkoxy groups containing from 1 to 10 carbon atoms, and mixtures thereof.

7. Device according to any one of the preceding claims, in which the volatile solvent is water or an organic solvent chosen from ethanol, isopropanol, acetone, isododecane, decamethylcyclopentasiloxane, octamethyltrisiloxane and decamethyltetrasiloxane, or mixtures thereof.

8. Device according to any one of the preceding claims, in which the treatment composition (P) comprises at least one hybrid acrylic hydrophobic film-forming polymer synthesized from at least one monomer bearing at least one (meth)acrylic acid group and/or esters of these acid monomers and/or amides of these acid monomers and from at least one styrene compound, preferably at least one hybrid acrylic film-forming polymer chosen from styrene/acrylate copolymers, in particular a copolymer derived from the polymerization of at least one styrene monomer and of at least one C₁-C₁₀ alkyl acrylate monomer.

9. Device according to any one of the preceding claims, in which the treatment composition (P) comprises at least one block silicone copolymer.

10. Device according to the preceding claim, in which the block silicone copolymer is obtained by a chain extension reaction, in the presence of a catalyst, from at least:
- (a) one polysiloxane (i) having at least one reactive group and preferably one or two reactive groups per molecule; and
- (b) one organosilicone compound (ii) which reacts with the polysiloxane (i) by a chain extension reaction.

11. Device according to any one of the preceding claims, in which the hydrophobic film-forming polymer(s) are present in an active material content ranging from 0.1% to 40% by weight, preferably ranging from 0.1% to 30% by weight, preferably ranging from 0.5% to 20% by weight, preferentially ranging from 1% to 20% by weight and more preferentially ranging from 1% to 15% by weight relative to the total weight of the composition.

12. Device according to any one of the preceding claims, the treatment composition (P) comprising one or more pigments.

13. Device according to any one of the preceding claims, the treatment composition (P) comprising an odorous compound or a mixture of odorous compounds, such as a perfume.

14. Device according to any one of the preceding claims, the treatment composition (P) comprising at least one mineral thickener, preferably chosen from clays, preferably a smectite.

15. Process for treating keratin fibres, comprising the application to the fibres of a treatment composition (P) contained in a device according to any one of the preceding claims, optionally followed by drying of the fibres.

## Patentansprüche

1. Vorrichtung (1) zum Aufbringen einer Keratinfaserbehandlungszusammensetzung, umfassend:
v) ein Applikatormittel (2), das fähig ist, eine Menge der Behandlungszusammensetzung (P) in einem Behälter (20) zurückzuhalten,
vi) ein Halteelement (4), das während einer Längsbewegung der Vorrichtung (1) relativ zu einer Strähne von Keratinfasern das Applikatormittel (2) fassen kann, um die Strähne von dem Applikatormittel (2) gefasst zu halten, um das Bestreichen der Strähne mit der Behandlungszusammensetzung (P) zu ermöglichen,
vii) wobei das Applikatormittel (2) eine Applikatorspitze (30) umfasst, die auf dem Behälter (20) befestigt ist und eine Auslassöffnung (31) umfasst, die mit einem Öffnungs/Verschlusselement (32) versehen ist, das in einer ersten Position die Auslassöffnung verschließt und in einer zweiten Position die Auslassöffnung wenigstens teilweise freigibt, wobei der Übergang von der ersten zu der zweiten Position als Reaktion auf eine Spannung erfolgt, die auf das Öffnungs/Verschlusselement (32) ausgeübt wird, indem die Strähne zwischen dem Halteelement und diesem Öffnungs/Verschlusselement (32) gefasst wird;
viii) wobei die Behandlungszusammensetzung (P) wenigstens ein hydrophobes filmbildendes Polymer und wenigstens ein flüchtiges Lösungsmittel umfasst.

2. Vorrichtung gemäß dem vorstehenden Anspruch, **dadurch gekennzeichnet, dass** das Halteelement (4) relativ zu dem Applikatormittel (2) beweglich ist.

3. Vorrichtung gemäß einem der vorstehenden Ansprüche, **dadurch gekennzeichnet, dass** das Halteelement (4) und das Applikatormittel (2) zwei getrennte Stücke bilden oder alternativ dazu über ein Verbindungsmittel (50) verbunden sind, um ein einziges Stück zu bilden.

4. Vorrichtung gemäß einem der vorstehenden Ansprüche, **dadurch gekennzeichnet, dass** die Applikatorspitze (3) eine Oberfläche (37) umfasst, die fähig ist, die Behandlungszusammensetzung (P) zu speichern und freizugeben.

5. Vorrichtung gemäß einem der vorstehenden Ansprüche, wobei das hydrophobe filmbildende Polymer ausgewählt ist aus Polymeren oder Copolymeren auf der Grundlage von Polyurethan, Polyacrylat, Vinylester-Copolymeren, Siliconharzen, Polyharnstoff/Polyurethan-Siliconen, Copolymeren auf der Grundlage von Siliconharz und von Dimethiconol, und reaktionsfähigen Siliconen, vorzugsweise hybriden Acrylatpolymeren.

6. Vorrichtung gemäß einem der vorstehenden Ansprüche, wobei das flüchtige Lösungsmittel Wasser oder ein organisches Lösungsmittel ausgewählt ist aus flüchtigen C₁-C₄-Alkanolen, flüchtigen C₅-C₇-Alkanen, flüchtigen Ölen auf Kohlenwasserstoffbasis, die 8 bis 16 Kohlenstoffatome enthalten, insbesondere verzweigten C₈-C₁₆-Alkanen, flüchtigen Silicon-Lösungsmittel, einschließlich Siliconverbindungen mit niedriger Viskosität ausgewählt aus linearen oder cyclischen Siliconen, die 2 bis 7 Siliciumatome enthalten, wobei diese Silicone gegebenenfalls Alkyl- oder Alkoxygruppen umfassen, die 1 bis 10 Kohlenstoffatom enthalten, und Gemische davon.

7. Vorrichtung gemäß einem der vorstehenden Ansprüche, wobei das flüchtige Lösungsmittel Wasser oder ein organisches Lösungsmittel ausgewählt aus Ethanol, Isopropanol, Aceton, Isododecan, Decamethylcyclopentasiloxan, Octamethyltrisiloxan und Decamethyltetrasiloxan oder Gemischen davon ist.

8. Vorrichtung gemäß einem der vorstehenden Ansprüche, wobei die Behandlungszusammensetzung (P) wenigstens ein hybrides hydrophobes filmbildendes Acrylpolymer umfasst, synthetisiert aus wenigstens einem Monomer, das wenigstens eine (Meth)acrylsäuregruppe und/oder Ester dieser Säuremonomere und/oder Amide dieser Säuremonomere trägt, und wenigstens einer Styrolverbindung, vorzugsweise wenigstens ein hybrides filmbildendes Acrylpolymer ausgewählt aus Styrol/Acrylat-Copolymeren, insbesondere ein Copolymer abgeleitet aus der Polymerisation von wenigstens einem Styrol-Monomer und wenigstens einem C₁-C₁₀-Alkylacrylat-Monomer.

9. Vorrichtung gemäß einem der vorstehenden Ansprüche, wobei die Behandlungszusammensetzung (P) wenigstens ein Block-Silicon-Copolymer umfasst.

10. Vorrichtung gemäß dem vorstehenden Anspruch, wobei das Block-Silicon-Copolymer durch eine Kettenverlängerungsreaktion in Gegenwart eines Katalysators von wenigstens:
(a) einem Polysiloxan (i) mit wenigstens einer reaktionsfähigen Gruppe und vorzugsweise einer oder zwei reaktionsfähigen Gruppen pro Molekül; und
(b) einer Organosiliconverbindung (ii), die durch eine Kettenverlängerungsreaktion mit dem Polysiloxan (i) reagiert,
erhalten ist.

11. Vorrichtung gemäß einem der vorstehenden Ansprüche, wobei das hydrophobe filmbildende Polymer bzw. die hydrophoben filmbildenden Polymere mit einem Wirkstoffgehalt im Bereich von 0,1 Gew.-% bis 40 Gew.-%, vorzugsweise im Bereich von 0,1 Gew.-% bis 30 Gew.-%, vorzugsweise im Bereich von 0,5 Gew.-% bis 20 Gew.-%, vorzugsweise im Bereich von 1 Gew.-% bis 20 Gew.-% und bevorzugter im Bereich von 1 Gew.-% bis 15 Gew.-% bezogen auf das Gesamtgewicht der Zusammensetzung vorhanden ist.

12. Vorrichtung gemäß einem der vorstehenden Ansprüche, wobei die Behandlungszusammensetzung (P) ein oder mehrere Pigmente umfasst.

13. Vorrichtung gemäß einem der vorstehenden Ansprüche, wobei die Behandlungszusammensetzung (P) eine wohlriechende Verbindung oder ein Gemisch von wohlriechenden Verbindungen, wie z. B. ein Parfüm, umfasst.

14. Vorrichtung gemäß einem der vorstehenden Ansprüche, wobei die Behandlungszusammensetzung (P) wenigstens ein Mineralstoff-Verdickungsmittel, vorzugsweise ausgewählt aus Tonen, vorzugsweise einen Smektit, umfasst.

15. Verfahren zum Behandeln von Keratinfasern, umfassend das Aufbringen einer Behandlungszusammensetzung (P), die in einer Vorrichtung gemäß einem der vorstehenden Ansprüche enthalten ist, auf die Fasern, gegebenenfalls gefolgt von Trocknen der Fasern.

## Revendications

1. Dispositif (1) pour appliquer une composition de traitement de fibres de kératine, comprenant:
v) des moyens d'applicateur (2) qui sont capables de retenir une quantité de la composition de traitement (P) dans un récipient (20);
vi) un élément de support (4) qui peut s'engager avec les moyens d'applicateur (2) dans le but, pendant un déplacement longitudinal du dispositif (1) par rapport à un bouchon de fibres de kératine, de maintenir ledit bouchon en engagement avec les moyens d'applicateur (2) de manière à permettre au bouchon d'être revêtu avec la composition de traitement (P);
vii) lesdits moyens d'applicateur (2) comprennent une pointe d'applicateur (30) montée sur le récipient (20) et comportant un orifice de sortie (31) équipé d'une élément d'ouverture/fermeture (32) qui, dans une première position, ferme ledit orifice de sortie, et qui, dans une seconde position, dégage au moins partiellement ledit orifice de sortie, le passage de la première à la seconde position étant réalisé en réponse à une contrainte exercée sur l'élément d'ouverture/fermeture (32) en engageant ledit bouchon entre l'élément de support et cet élément d'ouverture/fermeture (32); et
viii) la composition de traitement (P) comprenant au moins un polymère filmogène hydrophobe et au moins un solvant volatil.

2. Dispositif selon la revendication précédente, **caractérisé en ce que** l'élément de support (4) est mobile par rapport aux moyens d'applicateur (2).

3. Dispositif selon l'une ou l'autre des revendications précédentes, **caractérisé en ce que** l'élément de support (4) et les moyens d'applicateur (2) forment deux pièces séparées ou sont alternativement reliés par l'intermédiaire de moyens de connexion (50) et forment une seule pièce.

4. Dispositif selon l'une quelconque des revendications précédentes, **caractérisé en ce que** la pointe d'applicateur (3) présente une surface (37) qui est capable de stocker et de libérer la composition de traitement (P).

5. Dispositif selon l'une quelconque des revendications précédentes, dans lequel le polymère filmogène hydrophobe est choisi parmi des polymères ou des copolymères basés sur le polyuréthane, le polyacrylate, des copolymères d'ester vinylique, des résines silicone, des silicones à base de polyurée/polyuréthane, des copolymères basés sur une résine silicone et sur le diméthiconol, et des silicones réactifs, de préférence des polymères d'acrylate hybrides.

6. Dispositif selon l'une quelconque des revendications précédentes, dans lequel le solvant volatil est l'eau ou un solvant organique choisi parmi des alcanols C₁-C₄ volatils, des alcanes C₅-C₇ volatils, des huiles basées sur des hydrocarbures volatils contenant de 8 à 16 atomes de carbone, et en particulier des alcanes C₈-C₁₆ ramifiés, des solvants silicone volatils incluant des composés de silicone à faible viscosité choisis parmi des silicones linéaires ou cycliques contenant de 2 à 7 atomes de silicium, ces silicones comprenant optionnellement des groupes alkyles ou alkoxy contenant de 1 à 10 atomes de carbone, ainsi que des mélanges de ceux-ci.

7. Dispositif selon l'une quelconque des revendications précédentes, dans lequel le solvant volatil est l'eau ou un solvant organique choisi parmi l'éthanol, l'isopropanol, l'acétone, l'isododécane, le décaméthylcyclopentasiloxane, l'octaméthyltrisiloxane et le décaméthyltétrasiloxane, ou des mélanges de ceux-ci.

8. Dispositif selon l'une quelconque des revendications précédentes, dans lequel la composition de traitement (P) comprend au moins un polymère filmogène hydrophobe acrylique hybride synthétisé à partir d'au moins un monomère portant au moins un groupe acide (méth)acrylique et/ou des esters de ces monomères d'acide et/ou des amides de ces monomères d'acide, et à partir d'au moins un composé de styrène, de préférence au moins un polymère filmogène acrylique hybride choisi parmi des copolymères de styrène/acrylate, en particulier un copolymère dérivé de la polymérisation d'au moins un monomère de styrène et d'au moins un monomère d'acrylate d'alkyle C₁-C₁₀.

9. Dispositif selon l'une quelconque des revendications précédentes, dans lequel la composition de traitement (P) comprend au moins un copolymère bloc de silicone.

10. Dispositif selon la revendication précédente, dans lequel le copolymère bloc de silicone est obtenu par une réaction d'extension de chaîne, en présence d'un catalyseur, parmi au moins:
(a) un polysiloxane (i) présentant au moins un groupe réactif et de préférence un ou deux groupe(s) réactif(s) par molécule; et
(b) un composé d'organosilicone (ii) qui réagit avec le polysiloxane (i) par une réaction d'extension de chaîne.

11. Dispositif selon l'une quelconque des revendications précédentes, dans lequel le(s) polymère(s) filmogène(s) hydrophobe(s) est (sont) présent(s) dans une plage de teneur en matière active s'étendant de 0,1 % en poids à 40 % en poids, de préférence s'étendant de 0,1 % en poids à 30 % en poids, de préférence s'étendant de 0,5 % en poids à 20 % en poids, de préférence s'étendant de 1 % en poids à 20 % en poids, et mieux encore s'étendant de 1 % en poids à 15 % en poids par rapport au poids total de la composition.

12. Dispositif selon l'une quelconque des revendications précédentes, dans lequel la composition de traitement (P) comprend un ou plusieurs pigment(s).

13. Dispositif selon l'une quelconque des revendications précédentes, dans lequel la composition de traitement (P) comprend un composé odorant ou un mélange de composés odorants, tel qu'un parfum.

14. Dispositif selon l'une quelconque des revendications précédentes, dans lequel la composition de traitement (P) comprend au moins un épaississant minéral, de référence choisi parmi des argiles, de préférence une argile smectique.

15. Procédé de traitement de fibres de kératine, comprenant l'application aux fibres de la composition de traitement (P) contenue dans un dispositif selon l'une quelconque des revendications précédentes, optionnellement suivie par un séchage des fibres.
